# EUROPEAN PATENT APPLICATION

(11) **EP 4 751 733 A2**
(43) Date of publication of application: **03.06.2026**
(21) Application number: 26172867.9
(22) Date of filing: 09.04.2021
(51) Int. Cl.: A61K 39/215

(54) **IMPROVED METHODS OF PRODUCING A LIPIDATED PROTEIN**

(30) Priority: 09.04.2020 EP 20169148; 09.04.2020 EP 20169147; 15.04.2020 EP 20169642; 22.07.2020 EP 20187283; 20.10.2020 EP 20202816
(62) Divisional of application: 21716471.4
(71) Applicant: Valneva Austria GmbH, 1030 Vienna (AT)
(72) Inventor: The designation of the inventor has not yet been filed
(74) Representative: Simmons & Simmons LLP (Munich)

(57) **Abstract**

The present invention relates to method of producing a lipidated protein, a pharmaceutical composition comprising the protein of any of SEQ ID NOs: 1, 2, and/or 3 and/or the lipidated form of a protein comprising the protein of SEQ ID NO: 7 (C-TAB.GS) and/or SEQ ID NO: 8 (C-TAB.G5.1), especially the protein of SEQ ID NO: 12 (Lip-C-TAB.G5.1), and/or a lipidated form of a protein comprising the protein of SEQ ID NO: 15 (Spike protein of SARS-CoV-2) and/or a lipidated form of a protein comprising the any of the proteins of SEQ ID NOs: 16-22 (hMPV F protein), and the pharmaceutical composition for use as a medicament, particularly a vaccine and/or for use in a method for eliciting an immune response in a human against Lyme disease, a disease caused by *Clostridium difficile* or hMPV and/or of SARS-CoV-2 (COVID-19).

## Description

### FIELD OF THE INVENTION

The present invention relates to method of producing a lipidated protein, a pharmaceutical composition comprising the protein of any of SEQ ID NOs: 1, 2, and/or 3 and/or the lipidated form of a protein comprising the protein of SEQ ID NO: 7 (C-TAB.G5) and/or SEQ ID NO: 8 (C-TAB.G5.1), especially the protein of SEQ ID NO: 12 (Lip-C-TAB.G5.1), and/or a lipidated form of a protein comprising the protein of SEQ ID NO: 15 (Spike protein of SARS-CoV-2) and/or a lipidated form of a protein comprising the any of the proteins of SEQ ID NOs: 16-22 (hMPV F protein), and the pharmaceutical composition for use as a medicament, particularly a vaccine and/or for use in a method for eliciting an immune response in a human against Lyme disease, a disease caused by *Clostridium difficile* or hMPV and/or of SARS-CoV-2 (COVID-19).

### BACKGROUND OF THE INVENTION

Protein lipidation is one of the major post-translational modifications of proteins. The attachment of the lipid moiety frequently determines the localization and the function of the lipoproteins. Lipidated proteins participate in many essential biological processes in eukaryotic cells, including vesicular trafficking, signal transduction, pathology and immune response. For proper activity, fully functional lipidated proteins are required.

The commercial use of lipidated proteins requires access to a reasonable amount of lipid-modified proteins with defined structures and functional groups. However, access to lipoproteins by means of standard laboratory expression is often rather limited. Upscaling production of lipidated proteins to obtain larger amounts of the proteins is frequently difficult, as it effects the lipidation profile of the protein, which may change the biological activity of the protein.

Accordingly, the present invention aims at providing scaled-up methods for producing lipidated proteins, particularly vaccines.

### SUMMARY OF THE INVENTION

During the course of developing a scaled-up fermentation process for recombinant lipidated proteins, it was observed that several factors influence the lipid profile of the proteins; i.e., the types of fatty acids added during post-translational modification. Factors include trace element concentration, pH, fermenter headspace pressure and the concentration of antifoaming agents. In this regard, the aim is to maintain lipid profile consistency in recombinant lipidated protein drug substances from batch to batch by controlling one or more of these parameters. This particularly applies to heterodimers of the Borrelia Outer surface protein A (OspA), F-proteins of human metapneumovirus (hMPV), the Spike protein of SARS-CoV-2 or *Clostridium difficile* toxin proteins, which may be used as a vaccine.

### BRIEF DESCRIPTION OF THE DRAWINGS

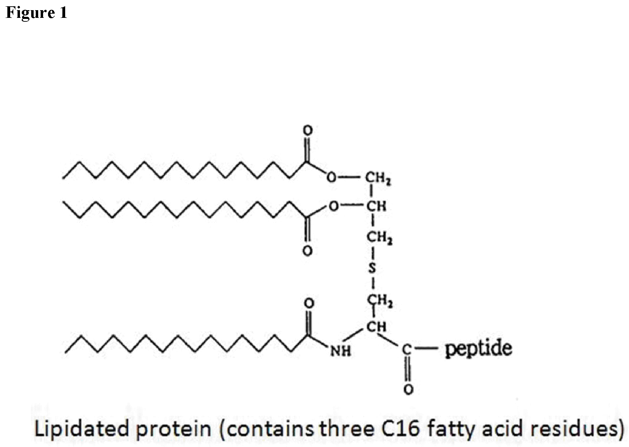

**Figure 1****.** An illustration of one possible lipid grouping on the lipidated OspA proteins disclosed herein. The shown grouping consists of three C16 fatty acids.

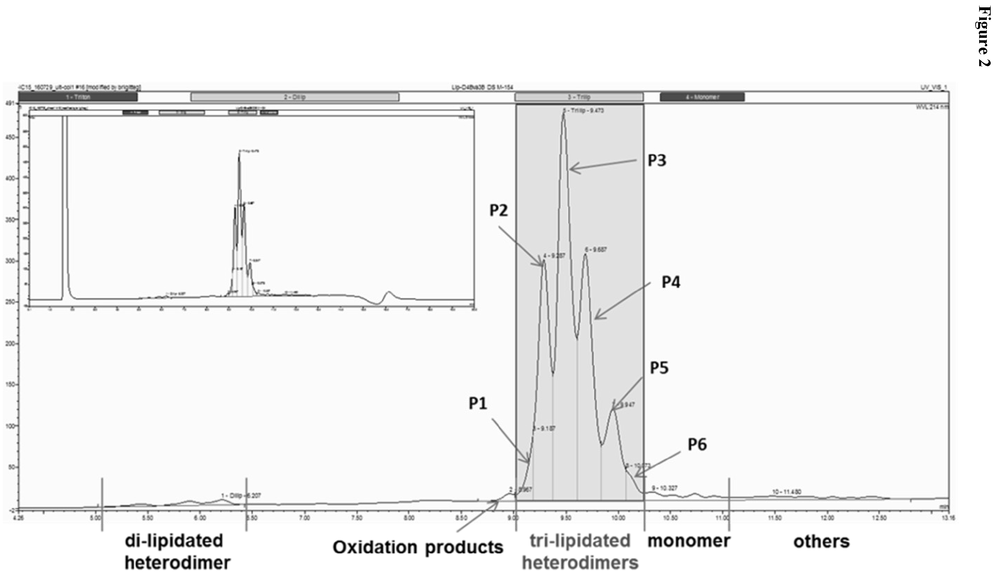

**Figure 2****.** Lipidation profile. Exemplary RP-HPLC chromatogram with peak assignment of Lip-S4D1-S3hybD1, GMP sample "M-154" (see also **Table 1).**

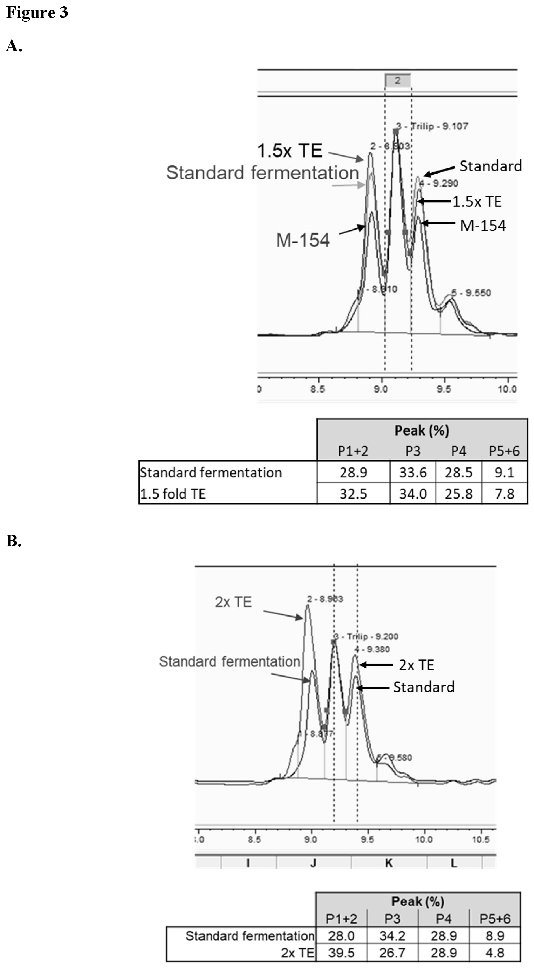

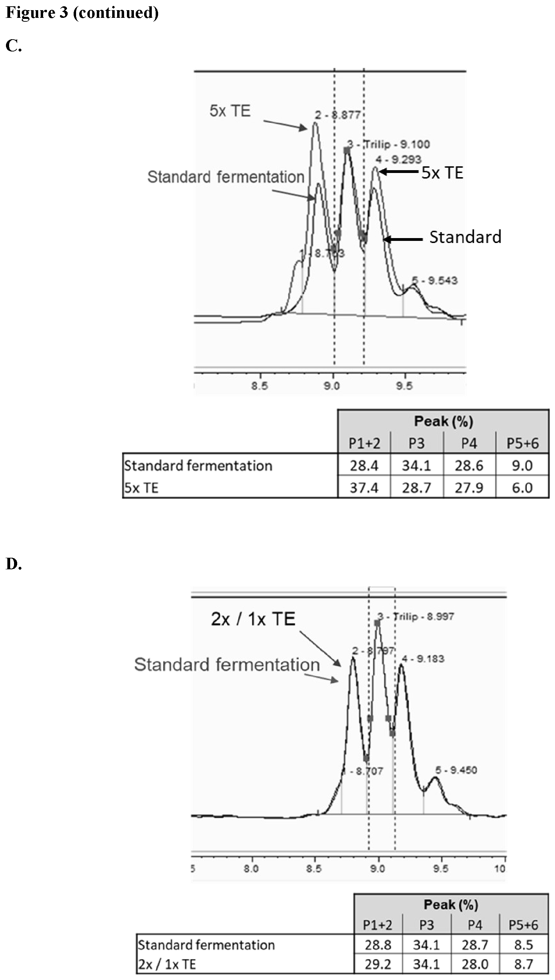

**Figure 3****.** Influence of added trace elements on protein lipidation profile. RP-HPLC chromatogram overlay of fermentation conditions for Lip-S4D1-S3hybD1 with **A)** a 1.5 fold increased TE solution, **B)** a 2 fold increased TE solution, **C)** a 5 fold increased TE solution, and **D)** a 2x TE solution in batch medium reduced to 1x during feed-phase, compared to standard fermentation conditions with the peak area in percentage (%). Peaks of the chromatogram are normalized for P3.

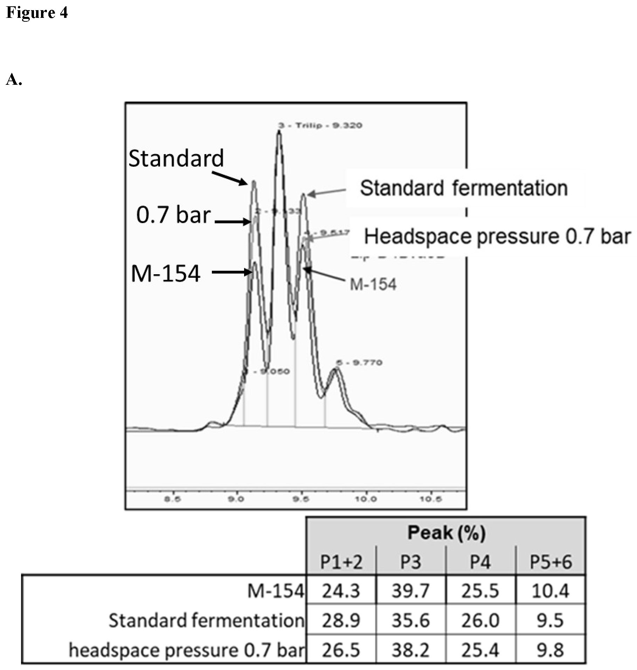

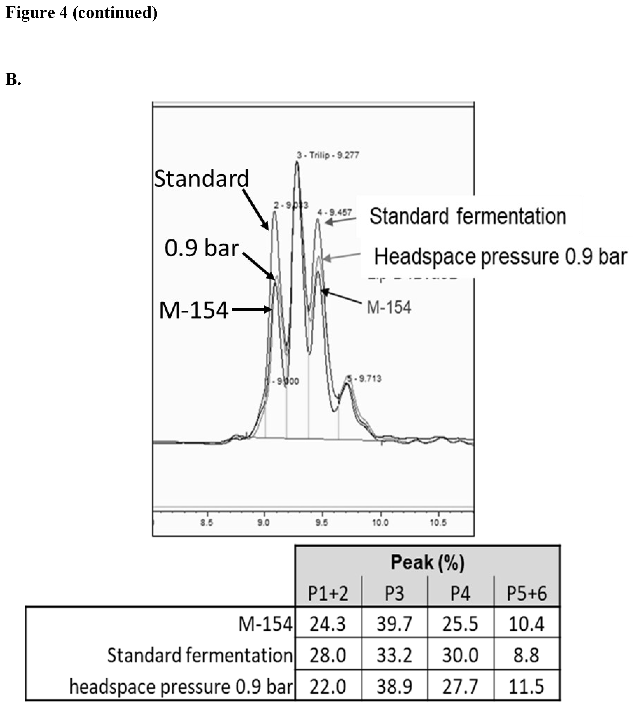

**Figure 4****.** Influence of applied pressure on protein lipidation profile. RP-HPLC chromatogram overlay of Lip-S4D1-S3hybD1 produced under fermentation conditions with **A)** 0.7 bar and **B)** 0.9 bar headspace pressure during feed phase. Data are compared to standard fermentation condition of Lip-S4D1-S3hybD1 with the peak area in percentage (%). Peaks of the chromatogram are normalized for P3.

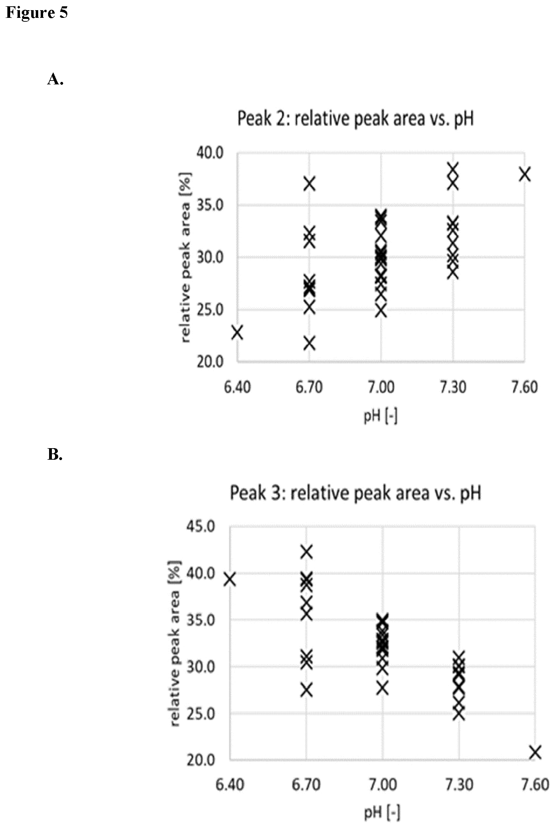

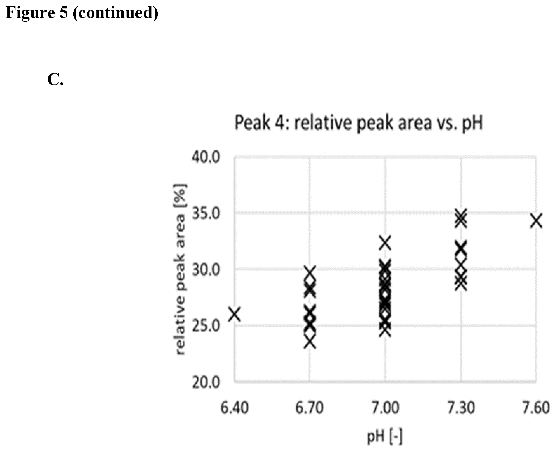

**Figure 5****.** Relative RP-HPLC peak area versus pH. Influence of pH on **A)** peak 2 (P2 i.e. P1+2), **B)** peak 3 (P3) and **C)** peak 4 (P4) of the characteristic lipid peak pattern of Lip-S1D1-S2D1. An aliquot of the biomass was used to prepare the Triton X-114 lipid phase and purified via hydroxyapatite.

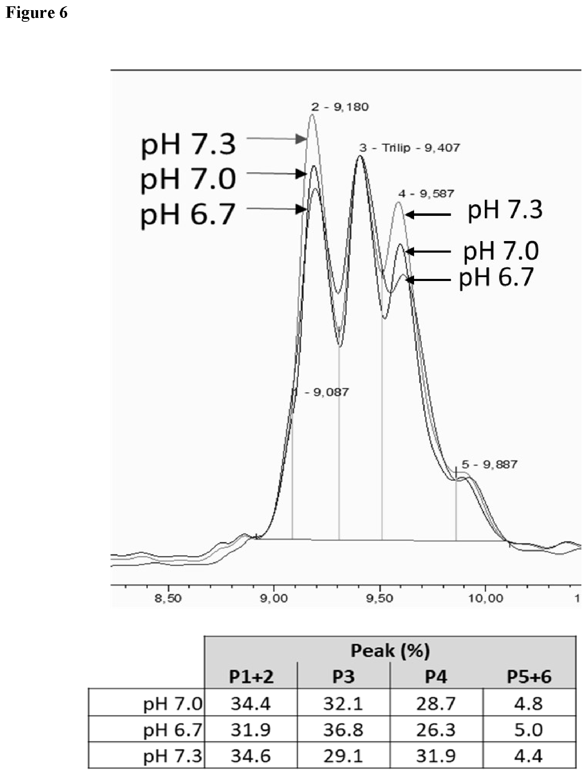

**Figure 6****.** Comparison of RP-HPLC peaks of a protein produced at three different pH levels. RP-HPLC chromatogram overlay of Lip-S4D1-S3hybD1 produced using fermentation conditions at pH 6.7, 7.0 and 7.3. Peaks of the chromatogram are normalized for P3. The individual peak area is shown in percentage (%) of the total area.

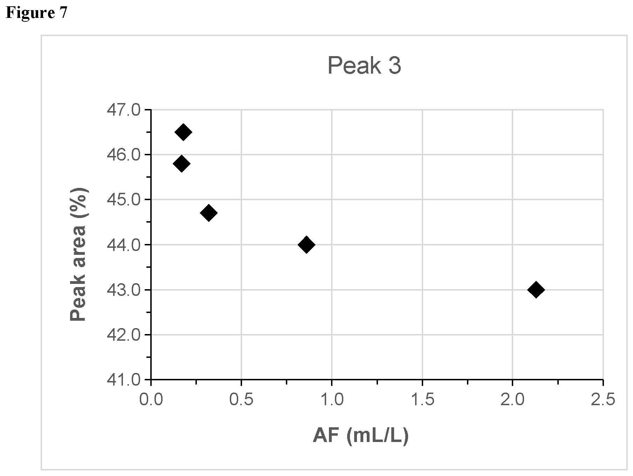

**Figure 7****.** Influence of anti-foam (AF) agent on protein lipidation profile. RP-HPLC analysis of fermentation conditions with antifoam addition on demand during feed-phase. The area of peak 3 in percentage (%) is plotted against the total amount of antifoam per fermenter volume (mL/L).

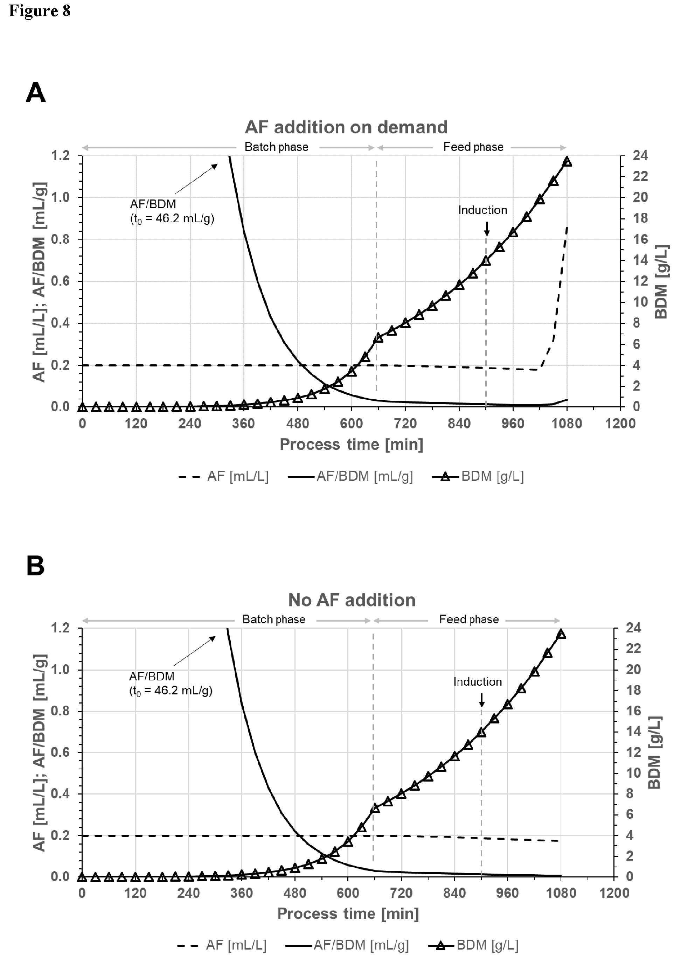

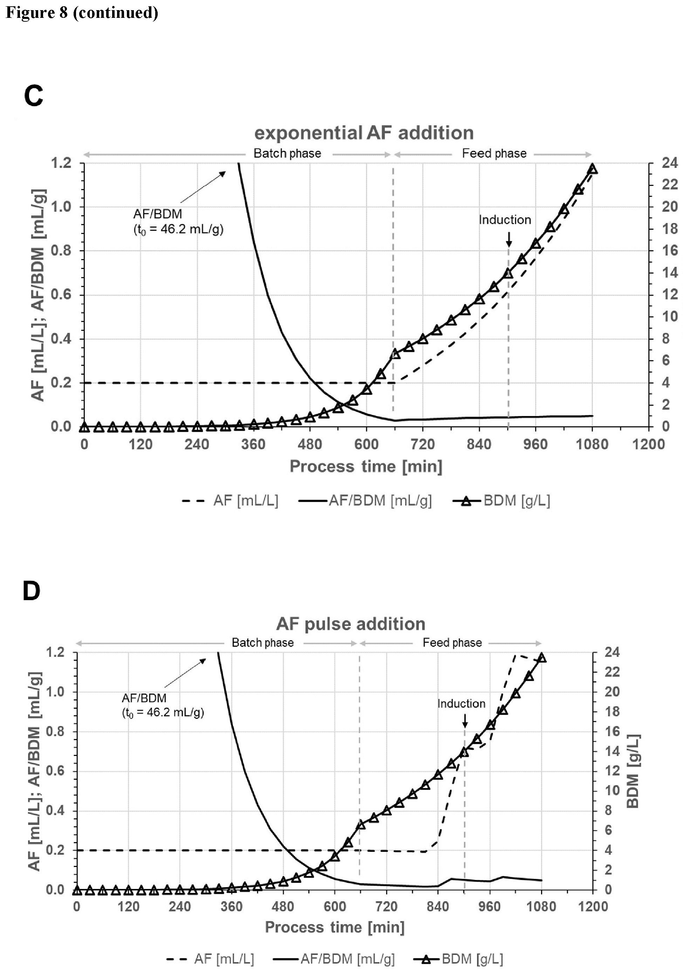

**Figure 8****.** The relationship of antifoam and calculated dry biomass (BDM) for a fed-batch fermentation process. **A)** AF addition on demand, **B)** no AF addition, **C)** AF added continuously with the exponential feed and **D)** AF pulse addition. Shown are the concentration of AF (dotted line), the calculated dry biomass (Δ) and the ratio of AF over biomass.

### DETAILED DESCRIPTION OF THE INVENTION

In order to provide a lipidated protein in an amount sufficient for commercial use, e.g. as a vaccine to be introduced to the market and used in health care, it has to be produced in large scale. However, upscaling production processes often results in structural changes of the product. When upscaling the production of lipidated OspA heterodimers, it was found that the lipidation profile of the OspA heterodimer may change depending on the conditions selected during the production in *E. coli* cells in a fed-batch process (see Examples).

Particularly, it has been found that fermenter headspace pressure and pH, optionally in combination with trace element concentration and the concentration of antifoaming agents are of relevance. This particularly applies to any subunit vaccine such as particular surface proteins (Spike protein of SARS-CoV-2), fusion proteins, e.g. heterodimers of the Borrelia Outer surface protein A (OspA), which may be used as a vaccine. It is well described in the literature that recombinant produced OspA proteins have a complex and heterogeneous lipid pattern that might depend on expression host and process conditions (Bouchon et al., 1997 Analytical biochemistry 246, 52-61). For more details about protein lipidation please refer to Nadolski et al, 2007, FEBS Journal 274, 5202-5210.

In a first aspect, the present invention relates to a method of producing a lipidated protein, wherein the lipidated protein has one to three lipids attached to a glycerol and the N-terminal cysteine of the protein, particularly wherein the lipidated protein has one lipid and a glycerol substituted with two lipids attached to the amino group of the N-terminal cysteine and/or particularly wherein the three acyl residues of the lipids are independently selected from C₁₄₋₂₀ alkyl and/or C₁₄₋₂₀ alkenyl, preferably wherein the lipidated protein has the formula (I): 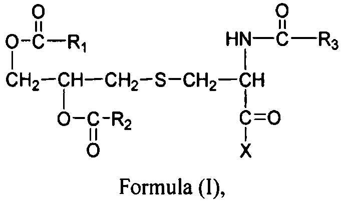 in which R₁, R₂ and/or R₃ are independently selected from C₁₄-C₂₀ alkyl or C₁₄-C₂₀ alkenyl and in which X is an amino acid sequence attached to the cysteine residue, the method comprising:
a) culturing *E coli* cells producing the lipidated protein in a volume of at least 40 L under a defined pressure and a defined pH;
b) harvesting the produced lipidated protein by extraction from *E. coli* cell culture, with e.g. a detergent, e.g. Triton X-114,
wherein the pressure and the pH are selected to obtain a lipidation profile of the lipidated proteins, in which about 40- 60 % of the fatty acids are palmitic acid (16:0), about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid (16:0), about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms.

Alternatively, the pressure and the pH are selected to obtain an RP-HPLC lipidation profile of the lipidated proteins, wherein a first peak (P1+P2) represents the Lip of formula (I) with two lipids being C16:0 and one being C16:1, a second peak (P3) represents the Lip of formula (I) with two lipids being C16:0 and one being C17:1, a third peak (P4) represents the Lip of formula (I) with two lipids being C16:0 and one being C18:1 and a fourth peak (P5+P6) represents the Lip of formula (I) with two lipids being C16:0 and one being cycC19, wherein peaks P1+P2, P3, P4 and P5+P6 comprise 23±10%, 41±10%, 25±10% and 12±10% of the total lipidated proteins, respectively. Preferably, the peaks P1+P2, P3, P4 and P5+P6 comprise 23±5%, 41±5%, 25±5% and 12±5% of the total lipidated proteins, respectively.

In one embodiment, step a) comprising culturing *E coli* cells producing a lipidated protein is separated into at least two phases: i) the batch phase and ii) the feed phase. The batch phase is defined as a phase of initial growth of *E. coli* following seeding of the large volume of medium in the fermenter e.g., from about 40L to up to about 2000L. The batch phase lasts for a period of several hours e.g., 8 to 24 hours, or up to about 12 hours. The feed phase is defined as the phase during which the recombinant protein is expressed as a result of induction, i.e., by the addition of and inducing agent, e.g. IPTG. The feed phase is typically shorter than the batch phase lasting for about e.g. between 3 and 8 hours, especially about 7 hours.

In accordance with the present invention, the lipidated protein or lipoprotein may be any naturally occurring or engineered protein, such as a fusion protein or a heterodimer, which has covalently attached one or more lipids. Preferably, the N-terminal amino acid of the protein is a cysteine and the lipidated protein comprises three lipids. The term "lipidated protein" refers to a protein that is not lipidated in its native form, but is modified, e.g., by adding a lipoprotein signal peptide, so that it is produced in lipidated form. Lipoprotein signal peptides (or lipid signal peptides), found on natural lipoproteins, are known in the art. Lipidation of a protein with an N-terminal lipidation signal sequence, such as those present on a nascent OspA polypeptide, occurs in the *E. coli* expression vector by the step-wise action of the enzymes diacylglyceryl transferase, signal peptidase II and transacylase, respectively. The first step is the transfer of a diacylglyceride to the cysteine sulphydryl group of the unmodified pro-protein, followed by the cleavage of the signal peptide by signal peptidase II and, finally, the acylation of the [alpha]-amino group of the N-terminal cysteine of the protein. The result is the placement of one lipid and a glycerol group substituted with two further lipids on the N-terminal cysteine residue of the polypeptide. The lipidation signal sequence, which is cleaved off during lipidation, is not present in the final polypeptide sequence.

According to the present invention, the lipidated protein has one, two or three lipids attached to a glycerol and the amino group of the N-terminal cysteine. The lipid moieties, along with the glycerol group of the lipidated protein, is also referred to as "Lip". Lip comprises one, two or three lipids, such as C₁₄₋₂₀ alkyl and/or C₁₄₋₂₀ alkenyl, attached to a glycerol and the N-terminal cysteine of the polypeptide of the invention, particularly wherein the lipidated protein has one lipid and a glycerol substituted with two lipids attached to the amino group of the N-terminal cysteine of the protein and/or particularly wherein the three acyl residues of the lipids are independently selected from C₁₄₋₂₀ alkyl and/or C₁₄₋₂₀ alkenyl. Preferably, Lip is a moiety of formula (I) below, 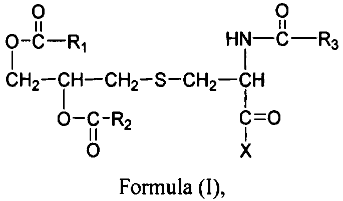 in which R₁, R₂ and/or R₃ are independently selected from C₁₄-C₂₀ alkyl or C₁₄-C₂₀ alkenyl and in which X is an amino acid sequence attached to the cysteine residue shown in Formula (I). More preferably, Lip plus the N-terminal cysteine of the polypeptide is N-palmitoyl-S-(2RS)-2,3-bis-(palmitoyloxy) propyl cysteine (referred to herein as "Pam3Cys") and is connected via the carbonyl C of the N-terminal cysteine to said amino acid sequence of the invention. In Formula (I) above R1, R2 and R3 would be palmitoyl moieties (16:0) and X is an amino acid sequence attached to the cysteine residue (see Fig. 1).

A suitable and exemplary method for determining the lipidation profile of a composition of the proteins of interest is described in the following, which has been carried out for the OspA protein in accordance with a preferred embodiment of the present invention. In order to determine the intended lipidation profile, various samples, including toxicological, engineering and GMP material, were analyzed by GC-FID for fatty acid composition of lipidated OspA proteins. After release of the fatty acids from the heterodimers and direct methylation (based on a method of O'Fallon et al., 2007, A direct method for fatty acid methyl ester synthesis: Application to wet meat tissues, oils and feedstuffs, J Anim Sci 2007.85:1511-1521), the methyl esters were analyzed on an Agilent 7890B gas chromatography system with FID. Separation was performed on a J&W HP-88 capillary GC column according to Agilent application note 5990-8429. Besides comparison of retention times with a reference standard (Supelco C8-C24), the identity of the individual detected fatty acids was confirmed by GC-MS. A summary of the respective fatty acid distribution in percent listed in Table A-1.

**Table A-1. Overview of fatty acid distribution (%) observed for toxicological (Tox), engineering (R) and GMP (M) material for each OspA antigen. Values were determined by Gas Chromatography with Flame-Ionization Detection (GC-FID).**

| **Fatty acid** | **Lip-S1D1-52D1 (SEQ ID NO: 1)** | | | **Lip-S4D1-S3hybD1 (SEQ ID NO: 2)** | | | **Lip-S5D1-S6D1 (SEQ ID NO: 3)** | | |
|---|---|---|---|---|---|---|---|---|---|
| | **Tox** | **R** | **M** | **Tox** | **R** | **M** | **Tox** | **R** | **M** |
| **C14:0** | 2.2 | 2.8 | 3.1 | 2.7 | 3.0 | 2.8 | 4.7 | 2.8 | 2.8 |
| **C15:0** | 0.4 | 0.8 | 0.6 | 1.6 | 0.8 | 0.6 | 1.4 | 0.7 | 0.6 |
| **C16:0** | 51.2 | 50.3 | 51.0 | 50.1 | 50.9 | 51.4 | 50.1 | 50.9 | 51.0 |
| **C16:1** | 16.4 | 12.7 | 10.2 | 12.7 | 11.4 | 9.1 | 11.5 | 10.8 | 8.5 |
| **C17:1** | 10.3 | 15.9 | 18.5 | 15.7 | 15.7 | 18.8 | 16.1 | 16.9 | 20.2 |
| **C18:1** | 18.6 | 15.0 | 13.1 | 14.5 | 15.5 | 13.3 | 13.5 | 15.1 | 12.7 |
| **cycC19** | 1.0 | 2.6 | 3.5 | 2.6 | 2.8 | 3.9 | 2.6 | 2.8 | 4.2 |

Accordingly, the lipidation profile to be maintained may be characterized as follows: about 50 % (e.g. 40- 60 %) of the fatty acids of the lipidation sites are palmitic acid (C16:0), about 10 to 20% are mono-unsaturated fatty acids comprising 17 C atoms (C17:1), about 10 to 20% are mono-unsaturated fatty acids comprising 18 C atoms (C18:1) (oleic acid), about 5 to 20 % (e.g. about 8 to 15%) are mono-unsaturated fatty acids comprising 16 C atoms (C16:1) (palmitoleic acid) and about 0 to 10% are other fatty acids, such as e.g. about 1 to 5% are cyclopropane-comprising fatty acids having 19 C atoms (cycC19) (lactobacillic acid). Other fatty acids such as C14:0 and C15:0 are present at an even lower amount. These results shown in Table A-1 confirm that OspA heterodimers are modified as predicted (tri-lipidated form). The results also indicate no significant differences of material derived from Tox, Engineering and GMP runs.

Detailed characterization of the lipidated proteins was also done by liquid-chromatography (RP-HPLC) and mass spectrometry (LC-MS). Separation was performed on a Zorbax 300SB-CN narrow bore column (2.1x150 mm, 5 µm; Agilent) in a water / acetonitrile gradient (0.1% formic acid) from 20 to 80% acetonitrile within 15 minutes (flow rate 0.2 mL/min, column temperature 60°C). The obtained mass spectra (Waters micromass ZQ, ESI-MS) were de-convoluted by MaxEnt software (Waters Corporation). A first peak (P1+P2) represented the Lip of formula (I) with 2 residues being C16:0 and one being C16:1, a second peak (P3) represented the Lip of formula (I) with 2 residues being C16:0 and one being C17:1, a third peak (P4) represented the Lip of formula I with 2 residues being C16:0 and one being C18:1 and a fourth peak (P5+P6) represented the Lip of formula I with 2 residues being C16:0 and one being cycC19, wherein peaks P1+P2, P3, P4 and P5+P6 cover about 15-25%, about 40-50%, about 20-25% and about 10-20% of the area under the curve, respectively (see e.g., **Fig. 2** and **Table 2).**

In a further production setting, which resulted also in a functional OspA heterodimer, the following RP-HPLC lipid pattern of the purified OspA proteins was obtained:

Run success criteria by RP-HPLC (prelim. target/range):

| | |
|---|---|
| P1+P2: | 23 ± 10% (absolute)* |
| P3: | 41 ± 10% (absolute)* |
| P4: | 25 ± 10% (absolute)* |
| P5+P6: | 12 ± 10% (absolute)* |

| | |
|---|---|
| No additional new peaks present and detectable. * Alternatively, the percentage deviation (range) may be lower such as e.g. ±9%, ±8%, ±7%, ±6% or most preferred ±5%. | |

Accordingly, the production conditions (pressure and pH optionally in combination with trace elements and anti-foam agent) may be selected to obtain a lipidation profile of the produced lipidated proteins, in which peaks P1+P2, P3, P4 and P5+P6 (as defined above) are in the range of about 23 ± 10%, about 41 ± 10%, about 25 ± 10% and about 12 ± 10%, respectively. Preferably, the peaks P1+P2, P3, P4 and P5+P6 comprise 23±5%, 41±5%, 25±5% and 12±5% of the total lipidated proteins, respectively.

An alternative run success criterion by RP-HPLC is the following:

| | |
|---|---|
| Pl+P2: | 18 ± 10% (absolute)* |
| P3: | 46 ± 10% (absolute)* |
| P4: | 20 ± 10% (absolute)* |
| P5+P6: | 16 ± 10% (absolute)* |

| | |
|---|---|
| No additional new peaks present and detectable. * Alternatively, the percentage deviation (range) may be lower such as e.g. ±9%, ±8%, ±7%, ±6% or most preferred ±5%. | |

Accordingly, the production conditions (pressure and pH optionally in combination with trace elements and antifoam agent) may be selected to obtain a lipidation profile of the produced lipidated proteins, in which peaks P1+P2, P3, P4 and P5+P6 (as defined above) are in the range of about 18 ± 10%, about 46 ± 10%, about 20 ± 10% and about 16 ± 10%, respectively. Preferably, the peaks P1+P2, P3, P4 and P5+P6 comprise 18±5%, 46±5%, 20±5% and 16±5% of the total lipidated proteins, respectively.

Critical cultivation parameters which have an influence on the lipidation pattern supporting activities were identified. The results indicate that pH has a significant influence on the distribution of the lipid peak pattern, especially on P1+P2, P3 and P4. For instance the characteristic peak pattern changed when the pH was higher during induction. The relative area of peak P1+P2 increases whereas the peak area of P3 decreases (see **Figures 5** and **6****).**

In addition to pH, the application of headspace pressure during cultivation to facilitate oxygen supply also has an impact on the lipidation pattern. Additional headspace pressure from 0.3 to 1.2 bar was tested. The lipidation pattern under pressure was observed to have a higher peak P3 and at the same time a lower peak intensity of P1+2 and P4 at added headspace pressure of both 0.7 and 0.9 bar **(****Fig. 4A** and **4B****,** respectively). For example, an increase of peak 3 (P3) intensity to approximately 40%, which is similar to GMP material, compared to fermentations without applying headspace pressure. The increase in P3 was observed to arise between 0.4 and 0.5 bar, with no effect seen at the lowest pressure tested (0.3 bar) (data not shown).

Furthermore, it was observed herein that trace elements added during cultivation have an impact on the lipidation patterns of recombinant proteins, with different concentrations resulting in different lipidation profiles. A trace element is a chemical element having a very low concentration or availability. The nutritional requirements of a bacterium (e.g. *E. coli*) are generally met with water, inorganic ions, small molecules, and macromolecules; however, trace elements also play a role in bacterial nutrition. Trace elements are metal ions required by certain cells in very small amounts. As a general rule, trace elements are required in such small amounts that it is often not necessary to add them to culture medium as nutrients, as they are provided as "contaminants" of water or other media components. Trace elements usually act as cofactors for essential enzymatic reactions in the cell. The usual cations that qualify as trace elements in bacterial nutrition are Mn, Co, Zn, Cu, and Mo (Todar, K; Todar's Online Textbook of Bacteriology; Nutrition and Growth of Bacteria, p.1, http://textbookofbacteriology.net/nutgro.html; accessed 11-Mar-2021). Iron (Fe) is present in higher amounts in bacteria and, while it is not considered a trace element per se, the environmental availability of Fe profoundly influences bacterial processes, such as, e.g., the expression of iron-requiring bacterial proteins (Andrews, SC et al. Bacterial iron homeostasis (2003) FEMS Microbiology Reviews 27:215-237). As such, for the purposes of the invention, Fe is considered as a trace element, i.e., is included in a trace element solution to supplement nutrition of bacteria during fermentation.

In a preferred embodiment of the present invention, a trace element (TE) solution (also referred to herein as trace element (TE) cocktail) is added during culturing step a), particularly during batch phase i) and/or feed phase ii). Trace elements, also called micronutrients, encompass any chemical element required by living organisms that is less than 0.1 percent by volume and are usually as part of a vital enzyme (a cell-produced catalytic protein). Preferably, the trace element (TE) solution comprises Fe, Co, Cu, Zn and/or Mo ions. In a preferred embodiment, the TE solution comprises the trace elements in the form of Iron(III)chloride hexahydrate, cobalt(II)chloride hexahydrate, copper(II)chloride dehydrate, zinc chloride and sodium molybdate dehydrate. In one embodiment, the TE solution further comprises boric acid and/or hydrochloric acid (HCl). Alternative salts of Fe, Co, Cu, Zn and Mo may be suitable as well. In a more preferred embodiment, the TE stock solution comprises 1.6 g/L Iron(III)chloride hexahydrate, 0.27 g/L cobalt(II)chloride hexahydrate, 0.127 g/L copper(II)chloride dehydrate, 0.2 g/L zinc chloride, 0.2 g/L sodium molybdate dihydrate, 0.05 g/L boric acid and 16.7 mL hydrochloric acid. The TE stock solution may be added to the medium at a dilution of 1/10000 to 1/10 (ml TE : ml culture medium). It has been found that higher amounts of trace elements may be needed in the feed phase rather than the batch phase. Accordingly, suitable dilutions in the feed phase, i.e., added to the feed phase medium, may be from 1/10 to 1/60, such as 1/12, 1/24, 1/36, 1/48 and 1/60. Particularly preferred in the feed phase are higher amounts of TE solution, i.e., dilutions of around 1/12, 1/24, 1/36 or 1/48. Suitable dilutions in the batch phase; i.e., added to the batch phase medium, may be from 1/10000 to 1/1600, such as 1/8000, 1/6400, 1/3200 and 1/1600, preferably around 1/8000, such as from 1/7500 to 1/8500. In general, the volume of the feed phase medium is approximately 15% of the volume of the batch medium. For example, in a lab scale fermentation run, the batch phase volume may be about 8L and the feed phase about 1.2L. Amounts of TE solution added for "1x" and "5x" TE concentrations are shown in Table A-2.

**Table A-2. An example of Batch and Feed medium in a lab scale fermentation with different amounts of added trace element solution according to the invention.**

| | Batch medium | Feed medium |
|---|---|---|
| Medium | 8L | 1.2L |
| "1x" TE | 1mL (1/8000) | 20mL (1/60) |
| "5x" TE | 5mL (1/1600) | 100mL (1/12) |

In a further preferred embodiment of the present invention, an anti-foam agent is present during culturing step a). An anti-foaming agent is a chemical additive that prevents the formation of foam in industrial process liquids. Foam occurs in bioprocesses due to the introduction of gases into the culture medium, and is further stabilised by proteins produced by organisms in the culture. In formats of larger scale, foaming is a problem that is particularly acute due to gassing used to maintain appropriate dissolved oxygen (DO) concentrations. Foaming can lead to reduced process productivity since bursting bubbles can damage proteins, result in loss of sterility if the foam escapes the bioreactor or lead to overpressure if a foam-out blocks an exit filter. To prevent the formation of foam, one or more anti-foam agents may be employed in the method of the present invention. Anti-foam agents can be classified as either hydrophobic solids dispersed in carrier oil, aqueous suspensions/emulsions, liquid single components or solids and may contain surfactants. Examples for suitable anti-foam agents include without limitation silicone oil (S184), polypropylene glycol (PPG), such as PPG-2000, silicone oil/PPG mixture, and an emulsion containing 10% S184. A particular preferred anti-foam agent is PPG-2000.

In one embodiment, the anti-foam agent may be present during both i) batch phase and ii) feed phase. The anti-foam agent is especially suitable during the exponential phase of *E. coli* growth (feed phase) of the culturing. Therefore, the anti-foam agent is preferably added and/or increased in concentration during the exponential phase of *E. coli* growth (feed phase). It has been found that repeated or continuous addition of the anti-foam agent is particularly useful in the production of the lipidated proteins with the method of the present invention. Accordingly, the anti-foam agent is added repeatedly during culturing, especially in a bolus twice during the feed phase, preferably once before induction and once after induction. Alternatively, the anti-foam agent is added continuously during the feed phase (exponentially). In one embodiment, the anti-foam agent is present in both the batch phase and feed phase media.

Moreover, it has been found that the presence of an anti-foam agent during the course of fermentation can have an influence on the lipidation profile. The amount of antifoam per fermenter volume as well as the mode of addition has an impact on the peak area of peak 3 **(****Figures 7** and **8****).** Optimization of the amount of AF and the time and mode of administration can improve batch-to-batch consistency, which is crucial for bioprocess production scale.

Accordingly, the cultivation parameters pH and headspace pressure, optionally in combination with trace elements and/or an anti-foam agent, may be used to modulate the lipid peak pattern of recombinantly expressed lipidated OspA heterodimers in order to obtain the indicated lipidation pattern,
in which about 40- 60 % of the fatty acids are palmitic acid (16:0), about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms, or
in which 23±10% of the total lipidated proteins comprise two palmitic acids (16: 0) and one C16:1 fatty acid, 41±10% of the total lipidated proteins comprise two palmitic acids (16: 0) and one and one C17:1 fatty acid, 25±10% of the total lipidated proteins comprise two palmitic acids (16: 0) and one C18:1 fatty acid and 12±10% of the total lipidated proteins comprise two palmitic acids (16: 0) and one cycC19 fatty acid; or
in which 18±10% of the total lipidated proteins comprise two palmitic acids (16: 0) and one C16:1 fatty acid, 46±10% of the total lipidated proteins comprise two palmitic acids (16: 0) and one and one C17:1 fatty acid, 20±10% of the total lipidated proteins comprise two palmitic acids (16: 0) and one C18:1 fatty acid and 16±10% of the total lipidated proteins comprise two palmitic acids (16: 0) and one cycC19 fatty acid.

In a first step of the method of the present invention, the *E coli* cells producing the lipidated protein are cultured in a volume of at least 40 L under a defined pressure and a defined pH. The lipidated protein is produced by culturing *E. coli* cells producing a lipidated protein in a volume of at least 40 L under a defined pressure and a defined pH. In general, the fermentation process consists of inoculation of the synthetic batch medium with a pre-culture, a batch- and feed-phase where biomass generation and product formation occurs during feed-phase. The main fermenter may be completed in less than 20 h with a yield of wet cell biomass of e.g. approximately >90 g/L.

The lipidated protein is expressed in a host cell, namely an *E. coli* cell suitable for producing the protein in lipidated form, via conventional recombinant technology. Briefly, a DNA fragment encoding the protein is provided. The DNA fragment may be inserted into an E. *coli* expression vector to produce an expression plasmid. The expression plasmid may be introduced into a selected *E. coli* strain by transformation with the plasmid encompassing a nucleic acid sequence coding for the protein of interest (e.g. vector pET28b(+)) to allow for the production of the lipidated prortein, such as a OspA heterodimer. Transformation may be by heat shock. Positive transformants are cultured under suitable conditions for protein expression. Suitable cells may be *E. coli* BL21(DE3), Genotype F⁻*ompT hsdSB*(rB⁻mB⁻) *gal dcm* (DE3) (Invitrogen). The lipidated protein thus expressed can be isolated from the *E. coli* cells and its lipidation status may be confirmed via methods known in the art, e.g., immunoblotting with an anti-lipoprotein antibody or mass spectrometry.

Cells may be cultured for a time and under conditions allowing for the production of the lipidated protein. The minimal volume in which the cells are cultured is 40 L. In a further preferred embodiment, the volume is at least 100 L, at least 200 L or at least 300 L. The maximal volume may be 2000 L or 1000 L.

As detailed above, the pH and pressure will be selected to obtain the intended lipidation profile. In a preferred embodiment, the amount of trace elements and/or anti-foam agents are also defined during culturing. Throughout the cultivation the dissolved oxygen level (DO) will usually be maintained at a constant level. The process may be monitored by in-process controls for several fermenter parameters like temperature, pH, DO, aeration rate, agitation rate, feeding rate, acid/base consumption and headspace pressure.

In a preferred embodiment, the pressure is a head space pressure of from 0-1 bar, more preferably from 0 to 0.9 bar, even more preferably from 0.4 to 0.9 bar. In an alternative or additional embodiment, the pH is in the range of about 6.7 to 7.3, more preferably about pH 7 (e.g. 7 ± 10 % or 7 ± 5 % or 7 ± 2 %). Additionally, the volume during cultivation is at least 100 L, at least 200 L or at least 300 L.

In a second step of the method of the present invention, the produced lipidated protein is harvested by extraction from *E. coli* cell culture, with e.g. a detergent, such as Triton X-114. For this, cells may be broken, e.g. by resuspending in lysis buffer and/or disrupting by high pressure homogenization (e.g. two passages at 800 bar). The lipid moiety of the protein may be utilized to selectively extract the proteins with detergent, such as Triton X-114. During solubilization the nonionic detergent replaces most lipid molecules in contact with the hydrophobic domain or lipid moiety and leads to the formation of a soluble protein-detergent mixed micelle. As the temperature is raised, the micellar molecular weight increases, and the solution turns suddenly turbid (cloud point). At this temperature a microscopic phase separation of the solution caused by formation of larger micelle aggregates occurs. These larger micelle aggregates become immiscible with water and start to separate from the water phase. This phase separation occurs until two clear phases are formed. Hydrophilic proteins are recovered in the aqueous phase, whereas hydrophobic proteins are enriched in the detergent phase after separation. The obtained proteins may be further purified as known in the art (e.g. extraction, chromatographic methods, ultrafiltration, etc.). Finally, the purified protein may be stored in a suitable solution (e.g. isotonic saline comprising excipients or stabilizers, pH 6.2 to 7.2, optionally comprising aluminum as an adjuvant) until use.

Preferably, the lipidated protein in accordance with the present invention is a vaccine. Exemplary protein vaccines include cholera toxin B as a component of Dukoral, chemically inactivated diphtheria toxin, *Clostridium difficile* toxin protein(s), particularly toxin A-toxin B fusion proteins as e.g. described as C-TAB.G5.1 (SEQ ID NO: 4) in WO 2012/028741 A1, hepatitis B surface antigen, SARS-CoV-2 surface antigen, particularly the Spike protein, inactivated tetanus toxin, hMPV F protein and Borrelia OspA proteins and variants thereof. Particularly, the Borrelia OspA protein is an OspA protein, especially an OspA heterodimer.

Lyme borreliosis (LB) is an emerging infectious disease transmitted by ticks in the northern hemisphere. Different vaccine candidates based on the Outer surface protein A (OspA) of *Borrelia* species have been developed, which are referred to as OspA protein in accordance with the present invention. As monovalent OspA-based vaccines precludes efficient protection with a vaccine based on OspA from only a single serotype due to heterogeneity in OspA sequences across different serotypes in Europe and elsewhere, chimeric OspA molecules have been developed. Chimeric OspA molecules comprising the proximal portion from one OspA serotype, together with the distal portion form another OspA serotype, while retaining antigenic properties of both of the parent polypeptides, may be used in the vaccination of Lyme disease or borreliosis. Such OspA proteins are described in WO2011/143617 and WO2011/143623. Moreover, it was found that the introduction of at least one disulfide bond in mutant fragments of OspA increases the protective capacity of the polypeptide comprising the mutant OspA fragment relative to a polypeptide comprising the wild-type OspA fragment (WO2014/006226). Moreover, hybrid C-terminal OspA fragments have been developed, wherein the hybrid fragment consists of a C-terminal domain of an OspA protein of Borrelia that is comprised of a fragment derived from an OspA protein of a Borrelia strain different than *B. garinii,* strain PBr, and a second fragment of OspA from *B. garinii,* strain PBr, and differs from the corresponding wild-type sequence at least by the introduction of at least one disulfide bond. (WO2015/104396). Further, immunogenic polypeptides comprising a stabilized C-terminal OspA domain comprising two or more specific OspA epitopes each from distinct *Borrelia* strains causing Lyme borreliosis and being able to induce a protective immune response to all of said distinct *Borrelia* strains have been provided (WO2018/189372). Particularly, any of the OspAs provided in the above applications may be used as a vaccine antigen in the context of the present invention. Particularly preferred is the previously-described multimeric *Borrelia* OspA vaccine, in which the C-terminal parts of two monomers of the six OspA serotypes (ST1 to ST6) stabilized with disulfide bonds were linked together in each of the three fusion proteins (further details are also given below). Preferably, the OspA protein is lipidated.

*Clostridium difficile* is the leading cause of nosocomial antibiotic associated diarrhea and has become a major health problem in hospitals, nursing home and other care facilities. C. *difficile* associated disease (CDAD) is induced by the disruption of the normal colonic flora, usually the result of the administration of antibiotics. Following exposure to *C. difficile* spores in the environment, the organism may colonize the intestinal mucosa where the production of disease causing toxins can result in CDAD. Disease may range from mild uncomplicated diarrhea to severe pseudomembranous colitis and toxic megacolon. CDAD is the result of the actions of two exotoxins produced by C. *difficile,* toxin A and toxin B (also referred to as CTA and CTB, respectively). Both toxins are high molecular weight (~300 kDa) secreted proteins that possess multiple functional domains (Voth DE and Ballard JD, Clinical Microbiology Reviews 18:247-263 (2005)). The N-terminal domain of both toxins contains ADP-glucosyltransferase activity that modifies Rho-like GTPases. This modification causes a loss of actin polymerization and cytoskeletal changes resulting in the disruption of the colonic epithelial tight junctions. This leads to excessive fluid exudation into the colon and a resulting diarrhea. The central domain contains a hydrophobic domain and is predicted to be involved in membrane transport. The C-terminal domain of both toxins contain multiple homologous regions called repeating units (RUs) that are involved in toxin binding to target cells (Ho et al, (2005) PNAS 102(51):18373-18378). The repeating units are classified as either short (21-30 amino acids) or long (~50 amino acids). Repeating units combine to form clusters, each usually containing one long and 3 - 5 short repeating units. The full-length toxin A possesses 39 repeating units (ARUs) organized into 8 clusters (Dove et al. Infect. Immun. 58:480-488 (1990), while the full-length toxin B contains 24 repeating units (BRUs) organized into 5 clusters (Barroso et al., Nucleic Acids Res. 18:4004 (1990); Eichel-Streiber et al., Gene 96:107-113 (1992)). Further details on suggested *Clostridium difficile* toxin protein based vaccines may be found in WO2012028741A1 and EP2753352B2. In one embodiment, the *Clostridium difficile* toxin protein C-TAB.G5.1 (SEQ ID NO: 4, as described in WO 2012/028741 A1) is lipidated as described herein.

SARS-CoV-2 was detected for the first time in China around November 2019. Since then, the virus has caused a global pandemic. The natural reservoir are bats and the virus belongs to the Coronaviridae family, genus Betacoronavirus (betaCoV). The virus has a ssRNA genome composed of 29,903 bp (Wuhan-Hu-1: Genbank Reference sequence: NC_045512.2), which encode a 9,860 amino acid polyprotein, comprising 25 non-structural proteins and 4 structural proteins: spike (S), envelope (E), membrane (M) and nucleocapsid (N) proteins. The spike protein is a particularly preferred target for a vaccine. SARS-CoV-2 presents a substantial public health threat. The Imperial College COVID-19 (disease caused by SARS-CoV-2) Response Team published in March 16, 2020, a report evaluating all possible methods available to stop or delay the spread of the virus, which could ultimately lead to the break-down of the healthcare system and hundreds of thousands of deaths in the UK alone. The report stated that only population-wide social distancing has a chance to reduce effects to manageable levels and these measures need to be followed until a vaccine is available. This recommendation would mean for most of the population quarantine for at least 18 months. The report concluded that a mass-producible vaccine is the only option to stop this pandemic, other than a willingness to sacrifice the elderly population. In view of the dramatic situation, there is an absolute urgent need for an effective vaccine against SARS-CoV-2 as fast as possible. Furthermore, various escape mutants have emerged (e.g. UK_B.1.1.7; South African_B.1.351; Californian_B.1.427/B.1.429 and Brazilian_P.1 variants) which further worsen the situation and thus addressing this unfortunate development needs to be addressed as well.

Human metapneumovirus (hMPV) is a leading cause of acute respiratory tract infections in young children (0 - 4 years), immunocompromised patients and in elderly that can be fatal for these categories of patients (Schildgen et al. 2011. Clinical Microbiology Reviews 24(4): 734-54). hMPV is an enveloped, single-stranded RNA virus of the genus Pneumovirus of the family Paramyxoviridae. The hMPV genome consists of eight genes encoding nine proteins, including three surface glycoproteins F, G and SH. Protection against hMPV is afforded mainly by neutralizing antibodies directed against the fusion (F) glycoprotein, which is highly conserved between different genotypes and shares similarities to other paramyxoviruses (see van den Hoogen et al. 2004. Emerging Infectious Diseases 10(4): 658-66; van den Hoogen et al. 2002. Virology 295(1): 119-32). Among several vaccination strategies investigated, a subunit vaccine containing a viral protein, especially the hMPV F protein, is the most promising (Melero & Mas. 2015. Virus Res. 209: 128-35). Paramyxoviral F protein is a type I integral membrane protein that spans the membrane once and contains at its N-terminus a signal peptide, which targets the ectodomain to the extracellular membrane. At the C-terminus, a hydrophobic stop-transfer domain (TM domain) anchors the protein in the membrane, leaving a short cytoplasmic tail. Further details on hMPV and suitable protein vaccines are derivable from WO 2020/234300 A1.

Preferably, the vaccine is an OspA protein. More preferably, the OspA protein is an OspA heterodimer. The OspA heterodimers are mutant OspA fragment heterodimers comprising disulfide-stabilized C-terminal OspA fragments and/or a hybrid C-terminal OspA fragment, wherein the hybrid fragment consists of a C-terminal domain of an OspA protein of *Borrelia* that is comprised of a fragment derived from an OspA protein of a *Borrelia* strain different than *B. garinii,* strain PBr, and a second fragment of OspA from *B. garinii,* strain PBr, and differs from the corresponding wild-type sequence at least by the introduction of at least one disulfide bond. The disulfide bonds of the C-terminal OspA fragments and the hybrid C-terminal fragment are disulfide bonds Type 1, e.g. cysteine residues are inserted at position 182 +/- 3 and 269 +/- 3 (for further details see WO2014/006226 and WO 2015/104396 A1). S3hyb indicates a fusion of amino acids 125-176 of *B. valaisiana* and amino acids 177-274 of *B. garinii,* strain PBr. Lip means lipidation and indicates the N-terminal addition of glycerol and fatty acid residues. The "LN1" peptide linker is a fusion of two separate loop regions of the N-terminal half of OspA *from B. burgdorferi* s.s., strain B31 (aa 65-74 and aa 42-53, with an amino acid exchange at position 53 of D53S) which has the following sequence: GTSDKNNGSGSKEKNKDGKYS (SEQ ID NO: 11).

Most preferably, the OspA heterodimer is the heterodimer protein of SEQ ID NO: 1 (LipS1D1-S2D1), the heterodimer protein of SEQ ID NO: 2 (Lip-S4D1-S3hybD1) and the heterodimer protein of SEQ ID NO: 3 (Lip-S5D1-S6D1).

Lip-S1D1-S2D1 is a heterodimer fusion protein of an OspA serotype 1 fragment and OspA serotype 2 fragment, each with a disulfide bond type 1, the heterodimer comprising an N-terminal CSS for addition of lipids, a LN1 linker sequence and an N-terminal lipidation. Amino acids 164-174 of the OspA serotype 1 fragment are replaced by non-hLFA-1-like sequence NFTLEGKVAND. The sequence is shown as follows:

Lip-S1D1-S2D1-aa SEQ ID NO: 1
LipCSSFNEKGEVSEKIITRADGTRLEYTGIKSDGSGKAKEVLKNFTLEGKVANDKTTLVV
KCGTVTLSKNISKSGEVSVELNDTDSSAATKKTAAWNSGTSTLTITVNSKKTKDLVFTKEN
TITVQQYDSNGTKLEGSAVEITKLDEICNALKGTSDKNNGSGSKEKNKDGKYSFNEKGELS
AKTMTRENGTKLEYTEMKSDGTGKAKEVLKNFTLEGKVANDKVTLEVKCGTVTLSKEIAKS
GEVTVALNDTNTTQATKKTGAWDSKTSTLTISVNSKKTTQLVFTKQDTITVQKYDSAGTNL
EGTAVEIKTLDELCNALK

Lip-S4D1-S3hybD1 is a heterodimer fusion protein of an OspA serotype 4 fragment and a hybrid OspA serotype 3 fragment, which hybrid comprises amino acids 125-176 of *B. valaisiana,* strain VS116 and amino acids 177-274 of *B. garinii,* strain PBr, serotype 3, each with a disulfide bond type 1, the heterodimer comprising an N-terminal CSS for addition of lipids, an LN1 linker sequence and N-terminal lipidation. The sequence is shown as follows:

Lip-S4D1-S3hybD1-aa SEQ ID NO: 2
LipCSSFNAKGELSEKTILRANGTRLEYTEIKSDGTGKAKEVLKDFALEGTLAADKTTLKV
TCGTVVLSKHIPNSGEITVELNDSNSTQATKKTGKWDSNTSTLTISVNSKKTKNIVFTKED
TITVQKYDSAGTNLEGNAVEIKTLDELCNALKGTSDKNNGSGSKEKNKDGKYSFNEKGEVS
EKILTRSNGTTLEYSQMTDAENATKAVETLKNGIKLPGNLVGGKTKLTVTCGTVTLSKNIS
KSGEITVALNDTETTPADKKTGEWKSDTSTLTISKNSQKTKQLVFTKENTITVQNYNRAGN
ALEGSPAEIKDLAELCAALK

Lip-S5D1-S6D1 is a heterodimer fusion protein of an OspA serotype 5 fragment and an OspA serotype 6 fragment, each with a disulfide bond type 1, the heterodimer comprising an N-terminal CSS for addition of lipids, LN1 linker sequence and an N-terminal lipidation. The sequence is shown as follows:

Lip-S5D1-S6D1-aa SEQ ID NO: 3
LipCSSFNEKGEISEKTIVRANGTRLEYTDIKSDKTGKAKEVLKDFTLEGTLAADGKTTLK
VTCGTVTLSKNISKSGEITVALDDTDSSGNKKSGTWDSGTSTLTISKNRTKTKQLVFTKED
TITVQNYDSAGTNLEGKAVEITTLKELCNALKGTSDKNNGSGSKEKNKDGKYSFNGKGETS
EKTIVRANGTRLEYTDIKSDGSGKAKEVLKDFTLEGTLAADGKTTLKVTCGTVVLSKNILK
SGEITAALDDSDTTRATKKTGKWDSKTSTLTISVNSQKTKNLVFTKEDTITVQRYDSAGTN
LEGKAVEITTLKELCNALK

The nucleic acid sequences encoding the above proteins are as follows:
**Lip-S1D1-S2D1-nt SEQ ID NO: 4** 
ATGAAAGCTACTAAACTGGTACTGGGCGCGGTAATCCTGGGTTCTACTCTGCTGGCAGGTT
GCTCAAGCTTCAACGAAAAGGGCGAAGTCAGCGAAAAAATCATTACCCGCGCAGACGGCAC
CCGCCTGGAATACACCGGCATCAAATCGGACGGCAGCGGCAAAGCGAAAGAAGTTCTGAAA
AACTTTACCCTGGAAGGCAAAGTCGCAAATGATAAAACCACCCTGGTGGTGAAATGCGGCA
CCGTTACGCTGAGCAAAAACATTAGTAAATCCGGTGAAGTCTCTGTGGAACTGAATGATAC
CGACAGCTCTGCGGCCACCAAGAAAACCGCAGCTTGGAACTCAGGCACCTCGACGCTGACC
ATTACGGTTAATAGCAAGAAAACCAAAGATCTGGTCTTCACGAAAGAAAACACCATCACGG
TGCAGCAATATGACAGCAATGGTACCAAACTGGAAGGCTCCGCTGTGGAAATCACGAAACT
GGATGAAATCTGTAATGCTCTGAAAGGTACTAGTGACAAAAACAATGGCTCTGGTAGCAAA
GAGAAAAACAAAGATGGCAAGTACTCATTCAACGAAAAAGGCGAACTGTCGGCGAAAACGA
TGACGCGTGAAAACGGCACCAAACTGGAATATACGGAAATGAAAAGCGATGGCACCGGTAA
AGCGAAAGAAGTTCTGAAAAACTTTACCCTGGAAGGCAAAGTCGCCAATGACAAAGTCACC
CTGGAAGTGAAATGCGGCACCGTTACGCTGTCAAAAGAAATTGCAAAATCGGGTGAAGTGA
CCGTTGCTCTGAACGATACGAATACCACGCAAGCGACCAAGAAAACCGGCGCCTGGGACAG
CAAAACCTCTACGCTGACCATTAGTGTTAATAGCAAGAAAACCACGCAGCTGGTCTTCACC
AAACAAGATACGATCACCGTGCAGAAATACGACAGTGCGGGTACCAACCTGGAAGGCACGG
CTGTTGAAATCAAAACCCTGGACGAACTGTGTAACGCCCTGAAA
**Lip-S4D1-S3hybD1-nt SEQ ID NO: 5** 
ATGAAAGCTACTAAACTGGTACTGGGCGCGGTAATCCTGGGTTCTACTCTGCTGGCAGGTT
GCTCAAGCTTCAATGCTAAGGGCGAACTGAGCGAAAAAACGATCCTGCGTGCGAATGGCAC
CCGTCTGGAATACACCGAAATCAAATCCGATGGTACGGGCAAAGCAAAGGAAGTCCTGAAA
GATTTTGCTCTGGAAGGTACCCTGGCGGCCGACAAAACCACGCTGAAGGTGACGTGCGGCA
CCGTGGTTCTGAGCAAACATATTCCGAACTCTGGTGAAATCACCGTTGAACTGAACGATAG
CAATTCTACGCAGGCAACCAAAAAGACGGGCAAATGGGACAGTAATACCTCCACGCTGACC
ATTTCAGTCAACTCGAAAAAGACCAAAAATATTGTGTTCACGAAGGAAGATACGATCACCG
TTCAAAAATATGACTCCGCGGGCACCAACCTGGAAGGCAATGCCGTCGAAATCAAAACCCT
GGATGAACTGTGTAACGCCCTGAAGGGTACTAGTGACAAAAACAATGGCTCTGGTAGCAAA
GAGAAAAACAAAGATGGCAAGTACTCATTCAACGAAAAAGGCGAAGTGAGCGAAAAAATTC
TGACCCGTAGCAATGGCACCACCCTGGAATATAGCCAGATGACCGATGCAGAAAATGCAAC
CAAAGCAGTTGAAACCCTGAAAAACGGTATTAAACTGCCTGGTAATCTGGTTGGTGGTAAA
ACCAAACTGACCGTTACCTGTGGCACCGTTACCCTGAGCAAAAACATTAGCAAAAGCGGTG
AAATTACCGTGGCACTGAATGATACCGAAACCACACCGGCAGACAAAAAAACCGGTGAATG
GAAAAGCGATACCAGCACCCTGACCATTAGTAAAAATAGCCAGAAAACAAAACAGCTGGTG
TTTACCAAAGAAAACACCATTACCGTGCAGAATTATAACCGTGCAGGTAATGCACTGGAAG
GTAGTCCGGCAGAAATTAAAGATCTGGCAGAACTGTGTGCAGCCCTGAAATAA
**Lip-S5D1-S6D1-nt SEQ ID NO: 6**

ATGAAAGCTACTAAACTGGTACTGGGCGCGGTAATCCTGGGTTCTACTCTGCTGGCAGGTT
GCTCAAGCTTCAACGAAAAGGGCGAAATCTCAGAAAAAACCATCGTCCGCGCTAACGGCAC
CCGCCTGGAATACACCGACATCAAATCAGACAAGACCGGTAAAGCGAAGGAAGTTCTGAAA
GATTTTACGCTGGAAGGTACCCTGGCAGCAGACGGTAAAACCACGCTGAAGGTGACCTGCG
GTACCGTTACGCTGTCCAAAAACATTAGTAAGTCCGGCGAAATCACGGTCGCCCTGGATGA
CACCGATAGCTCTGGCAACAAAAAGAGCGGTACCTGGGATTCAGGCACCTCGACGCTGACC
ATTTCTAAAAATCGTACGAAAACCAAGCAGCTGGTCTTCACGAAAGAAGATACGATCACCG
TGCAAAACTATGACAGCGCAGGTACCAATCTGGAAGGCAAAGCTGTGGAAATTACCACGCT
GAAAGAACTGTGTAATGCTCTGAAAGGTACTAGTGACAAAAACAATGGCTCTGGTAGCAAA
GAGAAAAACAAAGATGGCAAGTACTCATTCAACGGCAAAGGTGAAACGAGCGAAAAGACCA
TCGTGCGTGCGAACGGTACCCGCCTGGAATATACGGACATTAAATCGGACGGCAGCGGCAA

AGCAAAGGAAGTCCTGAAAGATTTTACGCTGGAAGGTACCCTGGCAGCAGACGGTAAAACC
ACGCTGAAGGTGACGTGCGGCACCGTGGTTCTGTCAAAAAACATTCTGAAGTCGGGTGAAA
TCACCGCAGCTCTGGATGACAGCGATACCACGCGTGCTACGAAAAAGACCGGTAAATGGGA
TAGCAAGACCTCTACGCTGACCATTAGTGTCAACTCCCAGAAAACGAAGAATCTGGTGTTC
ACCAAAGAAGATACGATCACCGTTCAACGCTATGACAGTGCGGGCACCAACCTGGAAGGCA
AAGCCGTTGAAATTACCACGCTGAAAGAACTGTGTAATGCTCTGAAA

Further information on the heterodimers and their production is derivable from WO 2015/104396 A1, wherein Lip-S1D1-S2D1, Lip-S4D1-S3hybD1 and Lip-S5D1-S6D1 correspond to SEQ ID NOs: 29, 27 and 33, respectively.

Also preferably, the protein is a *Clostridium difficile* toxin protein, particularly a *Clostridium difficile* toxin fusion protein. The *Clostridium difficile* toxin fusion protein comprises parts of toxin A fused to toxin B, particularly a parts of the C-terminal domain of toxin A fused to a part of the C-terminal domain of toxin B.

Most preferably, the vaccine comprises *Clostridium difficile* toxin fusion protein of SEQ ID NO: 7 (C-TAB.G5) and/or *Clostridium difficile* toxin fusion protein of SEQ ID NO: 8 (C-TAB.G5.1), more particularly it comprises the Clostridium difficile toxin fusion proteins in a weight ratio of 1:1 (C-TAB.G5 : C-TAB.G5.1).

The C-TAB.G5 or C-TAB.G5.1 comprises 19 repeating units of the C-terminal domain of toxin A fused to 23 repeating units of the C-terminal domain of toxin B. The present invention also includes compositions and formulations comprising the C-TAB.G5 or C-TAB.G5.1 isolated polypeptide. The sequences are shown as following SEQ ID NO: 7 and 8:
**C-TAB.G5 -aa SEQ ID NO: 7** 
MVTGVFKGPNGFEYFAPANTHNNNIEGQAIVYQNKFLTLNGKKYYFDNDSKAVTGWQTIDG
KKYYFNLNTAEAATGWQTIDGKKYYFNLNTAEAATGWQTIDGKKYYFNTNTFIASTGYTSI
NGKHFYFNTDGIMQIGVFKGPNGFEYFAPANTHNNNIEGQAILYQNKFLTLNGKKYYFGSD
SKAVTGLRTIDGKKYYFNTNTAVAVTGWQTINGKKYYFNTNTSIASTGYTIISGKHFYFNT
DGIMQIGVFKGPDGFEYFAPANTDANNIEGQAIRYQNRFLYLHDNIYYFGNNSKAATGWVT
IDGNRYYFEPNTAMGANGYKTIDNKNFYFRNGLPQIGVFKGSNGFEYFAPANTDANNIEGQ
AIRYQNRFLHLLGKIYYFGNNSKAVTGWQTINGKVYYFMPDTAMAAAGGLFEIDGVIYFFG
VDGVKAPGIYGRSMHNLITGFVTVGDDKYYFNPINGGAASIGETIIDDKNYYFNQSGVLQT
GVFSTEDGFKYFAPANTLDENLEGEAIDFTGKLIIDENIYYFDDNYRGAVEWKELDGEMHY
FSPETGKAFKGLNQIGDYKYYFNSDGVMQKGFVSINDNKHYFDDSGVMKVGYTEIDGKHFY
FAENGEMQIGVFNTEDGFKYFAHHNEDLGNEEGEEISYSGILNFNNKIYYFDDSFTAVVGW
KDLEDGSKYYFDEDTAEAYIGLSLINDGQYYFNDDGIMQVGFVTINDKVFYFSDSGIIESG
VQNIDDNYFYIDDNGIVQIGVFDTSDGYKYFAPANTVNDNIYGQAVEYSGLVRVGEDVYYF
 
GETYTIETGWIYDMENESDKYYFNPETKKACKGINLIDDIKYYFDEKGIMRTGLISFENNN
YYFNENGEMQFGYINIEDKMFYFGEDGVMQIGVFNTPDGFKYFAHQNTLDENFEGESINYT
GWLDLDEKRYYFTDEYIAATGSVIIDGEEYYFDPDTAQLVISE
**C-TAB.G5.1-aa SEQ ID NO: 8**

VTGVFKGPNGFEYFAPANTHNNNIEGQAIVYQNKFLTLNGKKYYFDNDSKAVTGWQTIDGK
KYYFNLNTAEAATGWQTIDGKKYYFNLNTAEAATGWQTIDGKKYYFNTNTFIASTGYTSIN
GKHFYFNTDGIMQIGVFKGPNGFEYFAPANTDANNIEGQAILYQNKFLTLNGKKYYFGSDS
KAVTGLRTIDGKKYYFNTNTAVAVTGWQTINGKKYYFNTNTSIASTGYTIISGKHFYFNTD
GIMQIGVFKGPDGFEYFAPANTDANNIEGQAIRYQNRFLYLHDNIYYFGNNSKAATGWVTI
DGNRYYFEPNTAMGANGYKTIDNKNFYFRNGLPQIGVFKGSNGFEYFAPANTDANNIEGQA
IRYQNRFLHLLGKIYYFGNNSKAVTGWQTINGKVYYFMPDTAMAAAGGLFEIDGVIYFFGV
DGVKAPGIYGRSMHNLITGFVTVGDDKYYFNPINGGAASIGETIIDDKNYYFNQSGVLQTG
VFSTEDGFKYFAPANTLDENLEGEAIDFTGKLIIDENIYYFDDNYRGAVEWKELDGEMHYF
SPETGKAFKGLNQIGDYKYYFNSDGVMQKGFVSINDNKHYFDDSGVMKVGYTEIDGKHFYF
AENGEMQIGVFNTEDGFKYFAHHNEDLGNEEGEEISYSGILNFNNKIYYFDDSFTAVVGWK
DLEDGSKYYFDEDTAEAYIGLSLINDGQYYFNDDGIMQVGFVTINDKVFYFSDSGIIESGV
QNIDDNYFYIDDNGIVQIGVFDTSDGYKYFAPANTVNDNIYGQAVEYSGLVRVGEDVYYFG
ETYTIETGWIYDMENESDKYYFNPETKKACKGINLIDDIKYYFDEKGIMRTGLISFENNNY
YFNENGEMQFGYINIEDKMFYFGEDGVMQIGVFNTPDGFKYFAHQNTLDENFEGESINYTG
WLDLDEKRYYFTDEYIAATGSVIIDGEEYYFDPDTAQLVISE

The nucleic acid sequences encoding the above proteins are as follows:
C-TAB.G5-nt SEQ ID NO: 9 
ATGCATAATTTGATAACTGGATTTGTGACTGTAGGCGATGATAAATACTACTTTAATCCAA
TTAATGGTGGAGCTGCTTCAATTGGAGAGACAATAATTGATGACAAAAATTATTATTTCAA
CCAAAGTGGAGTGTTACAAACAGGTGTATTTAGTACAGAAGATGGATTTAAATATTTTGCC
CCAGCTAATACACTTGATGAAAACCTAGAAGGAGAAGCAATTGATTTTACTGGAAAATTAA
TTATTGACGAAAATATTTATTATTTTGATGATAATTATAGAGGAGCTGTAGAATGGAAAGA
ATTAGATGGTGAAATGCACTATTTTAGCCCAGAAACAGGTAAAGCTTTTAAAGGTCTAAAT
CAAATAGGTGATTATAAATACTATTTCAATTCTGATGGAGTTATGCAAAAAGGATTTGTTA
GTATAAATGATAATAAACACTATTTTGATGATTCTGGTGTTATGAAAGTAGGTTACACTGA
AATAGATGGCAAGCATTTCTACTTTGCTGAAAACGGAGAAATGCAAATAGGAGTATTTAAT
ACAGAAGATGGATTTAAATATTTTGCTCATCATAATGAAGATTTAGGAAATGAAGAAGGTG
AAGAAATCTCATATTCTGGTATATTAAATTTCAATAATAAAATTTACTATTTTGATGATTC
ATTTACAGCTGTAGTTGGATGGAAAGATTTAGAGGATGGTTCAAAGTATTATTTTGATGAA
GATACAGCAGAAGCATATATAGGTTTGTCATTAATAAATGATGGTCAATATTATTTTAATG
ATGATGGAATTATGCAAGTTGGATTTGTCACTATAAATGATAAAGTCTTCTACTTCTCTGA
CTCTGGAATTATAGAATCTGGAGTACAAAACATAGATGACAATTATTTCTATATAGATGAT
AATGGTATAGTTCAAATTGGTGTATTTGATACTTCAGATGGATATAAATATTTTGCACCTG
CTAATACTGTAAATGATAATATTTACGGACAAGCAGTTGAATATAGTGGTTTAGTTAGAGT
TGGGGAAGATGTATATTATTTTGGAGAAACATATACAATTGAGACTGGATGGATATATGAT
ATGGAAAATGAAAGTGATAAATATTATTTCAATCCAGAAACTAAAAAAGCATGCAAAGGTA
TTAATTTAATTGATGATATAAAATATTATTTTGATGAGAAGGGCATAATGAGAACGGGTCT
TATATCATTTGAAAATAATAATTATTACTTTAATGAGAATGGTGAAATGCAATTTGGTTAT
ATAAATATAGAAGATAAGATGTTCTATTTTGGTGAAGATGGTGTCATGCAGATTGGAGTAT
TTAATACACCAGATGGATTTAAATACTTTGCACATCAAAATACTTTGGATGAGAATTTTGA
GGGAGAATCAATAAACTATACTGGTTGGTTAGATTTAGATGAAAAGAGATATTATTTTACA
GATGAATATATTGCAGCAACTGGTTCAGTTATTATTGATGGTGAGGAGTATTATTTTGATC
CTGATACAGCTCAATTAGTGATTAGTGAATAG
C-TAB.G5.1-nt SEQ ID NO: 10

CCATGGTTACAGGTGTTTTCAAAGGTCCGAACGGCTTTGAATATTTTGCACCGGCAAATAC
CCACAATAATAATATTGAAGGCCAGGCCATCGTGTATCAGAATAAATTTCTGACCCTGAAC
GGCAAAAAATACTATTTCGATAACGATAGCAAAGCAGTTACCGGTTGGCAAACCATTGATG
GCAAAAAATATTACTTCAACCTGAATACCGCAGAAGCAGCAACCGGCTGGCAGACGATCGA
CGGTAAAAAGTACTATTTTAACCTGAACACAGCCGAAGCCGCTACAGGCTGGCAGACAATA
GATGGGAAGAAGTATTATTTTAATACCAATACCTTTATTGCCAGCACCGGCTATACCAGCA
TTAATGGCAAACACTTCTATTTTAACACCGATGGTATTATGCAGATCGGTGTGTTTAAGGG
CCCTAATGGTTTTGAGTACTTCGCTCCGGCTAATACCGATGCAAATAACATCGAAGGTCAG
GCAATTCTGTACCAGAACAAATTTTTAACGCTGAACGGTAAGAAATATTACTTTGGTAGCG
ATTCAAAAGCCGTTACCGGTCTGCGTACGATCGACGGCAAGAAATATTATTTCAATACAAA
CACCGCAGTTGCCGTGACAGGTTGGCAGACGATAAATGGTAAGAAGTACTACTTCAACACC
AATACCAGCATTGCAAGTACCGGTTATACCATTATCAGCGGCAAACACTTTTACTTCAATA
CAGACGGCATTATGCAGATTGGCGTTTTCAAAGGTCCGGATGGTTTCGAGTACTTTGCCCC
TGCAAATACAGATGCAAACAATATTGAGGGACAGGCAATTCGCTATCAGAATCGTTTTCTG
TATCTGCACGATAACATCTATTACTTCGGCAATAATTCAAAAGCAGCCACCGGTTGGGTTA
CAATTGATGGTAATCGTTATTACTTTGAGCCGAATACCGCAATGGGTGCAAATGGTTATAA
AACCATCGATAACAAAAATTTTTATTTCCGCAACGGTCTGCCGCAGATTGGTGTTTTTAAG
GGTAGCAATGGCTTCGAGTATTTTGCGCCAGCCAACACCGATGCCAACAACATTGAAGGCC
AAGCGATTCGTTATCAAAACCGCTTTCTGCATCTGCTGGGCAAAATTTATTACTTTGGCAA
CAATAGCAAAGCGGTGACGGGCTGGCAAACCATTAACGGTAAAGTTTATTATTTCATGCCG
GATACCGCTATGGCAGCAGCCGGTGGTCTGTTTGAAATTGATGGCGTGATTTATTTTTTTG

GCGTGGATGGTGTTAAAGCACCGGGTATTTATGGTCGTAGCATGCATAATCTGATTACCGG
TTTTGTTACCGTGGGCGACGATAAATACTACTTTAATCCGATTAATGGTGGTGCAGCAAGC
ATTGGTGAAACCATTATCGATGACAAAAACTATTATTTTAACCAGAGCGGTGTTCTGCAGA
CAGGTGTTTTTAGCACCGAAGATGGCTTCAAATATTTTGCTCCTGCGAATACACTGGATGA
AAATCTGGAAGGTGAAGCAATTGATTTTACCGGCAAACTGATCATCGACGAGAACATCTAC
TATTTTGATGATAATTATCGCGGTGCCGTGGAATGGAAAGAACTGGATGGTGAAATGCACT
ATTTTAGTCCGGAAACCGGTAAAGCCTTTAAAGGTCTGAATCAGATCGGCGATTACAAGTA
TTACTTTAATTCAGATGGCGTGATGCAGAAAGGCTTTGTGAGCATTAACGACAACAAACAC
TATTTTGACGACAGCGGTGTGATGAAAGTGGGTTATACCGAAATCGACGGGAAACATTTTT
ATTTTGCCGAAAACGGCGAAATGCAGATTGGAGTATTTAATACCGAGGACGGCTTTAAATA
CTTTGCCCATCATAATGAAGATCTGGGTAATGAAGAAGGCGAAGAAATTAGCTATAGCGGC
ATTCTGAATTTTAATAACAAGATCTATTATTTCGATGATAGCTTCACCGCAGTTGTTGGTT
GGAAAGATCTGGAAGATGGCAGCAAATATTATTTTGATGAAGATACCGCAGAGGCCTATAT
TGGTCTGAGCCTGATTAATGATGGCCAGTATTATTTCAACGATGATGGTATCATGCAGGTT
GGTTTTGTGACCATCAACGATAAAGTGTTCTATTTCAGCGATAGCGGCATTATTGAAAGCG
GTGTTCAGAACATCGACGATAACTATTTCTACATCGATGATAACGGTATTGTTCAGATTGG
CGTGTTTGATACCTCCGATGGTTATAAATATTTCGCACCAGCCAATACCGTGAACGATAAT
ATTTATGGTCAGGCAGTTGAATATTCAGGTCTGGTTCGTGTTGGCGAAGATGTTTATTATT
TTGGCGAAACCTATACCATTGAAACCGGCTGGATCTATGATATGGAAAACGAGAGCGACAA
GTACTATTTCAATCCGGAAACGAAAAAAGCCTGCAAAGGCATTAATCTGATCGACGATATT
AAGTACTACTTTGACGAAAAAGGCATTATGCGTACCGGTCTGATTAGCTTTGAGAACAACA
ACTATTACTTCAATGAGAACGGTGAGATGCAGTTTGGCTATATCAACATCGAGGACAAAAT
GTTTTATTTTGGTGAGGACGGTGTGATGCAGATAGGGGTTTTTAATACACCGGATGGGTTT
AAGTATTTTGCACATCAGAACACCCTGGATGAAAACTTTGAAGGCGAAAGCATTAATTATA
CCGGTTGGCTGGATCTGGATGAGAAACGTTATTATTTCACCGACGAATACATTGCAGCAAC
CGGTAGCGTTATTATTGATGGTGAGGAATATTACTTCGATCCGGATACAGCACAGCTGGTT
ATTAGCGAATAACTCGAG

Further information on the proteins C-TAB.G5 and C-TAB.G5.1 and their production is derivable from WO 2012028741 A1 and EP2753352 B2, wherein C-TAB.G5 and C-TAB.G5.11 correspond to SEQ ID NOs: 2 and 4, respectively.

Further preferred lipidated *Clostridium difficile* toxin proteins according to the present invention include:
**Lip-C-TAB.G5.1 SEQ ID NO: 12** 
LipCSSFVTGVFKGPNGFEYFAPANTHNNNIEGQAIVYQNKFLTLNGKKYYFDNDSKAVTG
WQTIDGKKYYFNLNTAEAATGWQTIDGKKYYFNLNTAEAATGWQTIDGKKYYFNTNTFIAS
TGYTSINGKHFYFNTDGIMQIGVFKGPNGFEYFAPANTDANNIEGQAILYQNKFLTLNGKK
YYFGSDSKAVTGLRTIDGKKYYFNTNTAVAVTGWQTINGKKYYFNTNTSIASTGYTIISGK
HFYFNTDGIMQIGVFKGPDGFEYFAPANTDANNIEGQAIRYQNRFLYLHDNIYYFGNNSKA
ATGWVTIDGNRYYFEPNTAMGANGYKTIDNKNFYFRNGLPQIGVFKGSNGFEYFAPANTDA
NNIEGQAIRYQNRFLHLLGKIYYFGNNSKAVTGWQTINGKVYYFMPDTAMAAAGGLFEIDG
VIYFFGVDGVKAPGIYGRSMHNLITGFVTVGDDKYYFNPINGGAASIGETIIDDKNYYFNQ
SGVLQTGVFSTEDGFKYFAPANTLDENLEGEAIDFTGKLIIDENIYYFDDNYRGAVEWKEL
DGEMHYFSPETGKAFKGLNQIGDYKYYFNSDGVMQKGFVSINDNKHYFDDSGVMKVGYTEI
 
DGKHFYFAENGEMQIGVFNTEDGFKYFAHHNEDLGNEEGEEISYSGILNFNNKIYYFDDSF
TAVVGWKDLEDGSKYYFDEDTAEAYIGLSLINDGQYYFNDDGIMQVGFVTINDKVFYFSDS
GIIESGVQNIDDNYFYIDDNGIVQIGVFDTSDGYKYFAPANTVNDNIYGQAVEYSGLVRVG
EDVYYFGETYTIETGWIYDMENESDKYYFNPETKKACKGINLIDDIKYYFDEKGIMRTGLI
SFENNNYYFNENGEMQFGYINIEDKMFYFGEDGVMQIGVFNTPDGFKYFAHQNTLDENFEG
ESINYTGWLDLDEKRYYFTDEYIAATGSVIIDGEEYYFDPDTAQLVISE
Lip-ToxA-His SEQ ID NO: 13 
LipCSSFVTGVFKGPNGFEYFAPANTHNNNIEGQAIVYQNKFLTLNGKKYYFDNDSKAVTG
WQTIDGKKYYFNLNTAEAATGWQTIDGKKYYFNLNTAEAATGWQTIDGKKYYFNTNTFIAS
TGYTSINGKHFYFNTDGIMQIGVFKGPNGFEYFAPANTDANNIEGQAILYQNKFLTLNGKK
YYFGSDSKAVTGLRTIDGKKYYFNTNTAVAVTGWQTINGKKYYFNTNTSIASTGYTIISGK
HFYFNTDGIMQIGVFKGPDGFEYFAPANTDANNIEGQAIRYQNRFLYLHDNIYYFGNNSKA
ATGWVTIDGNRYYFEPNTAMGANGYKTIDNKNFYFRNGLPQIGVFKGSNGFEYFAPANTDA
NNIEGQAIRYQNRFLHLLGKIYYFGNNSKAVTGWQTINGKVYYFMPDTAMAAAGGLFEIDG
VIYFFGVDGVKAPGIYGLEHHHHHH
Lip-ToxB-His SEQ ID NO: 14

LipCSSFNLITGFVTVGDDKYYFNPINGGAASIGETIIDDKNYYFNQSGVLQTGVFSTEDG
FKYFAPANTLDENLEGEAIDFTGKLIIDENIYYFDDNYRGAVEWKELDGEMHYFSPETGKA
FKGLNQIGDYKYYFNSDGVMQKGFVSINDNKHYFDDSGVMKVGYTEIDGKHFYFAENGEMQ
IGVFNTEDGFKYFAHHNEDLGNEEGEEISYSGILNFNNKIYYFDDSFTAVVGWKDLEDGSK
YYFDEDTAEAYIGLSLINDGQYYFNDDGIMQVGFVTINDKVFYFSDSGIIESGVQNIDDNY
FYIDDNGIVQIGVFDTSDGYKYFAPANTVNDNIYGQAVEYSGLVRVGEDVYYFGETYTIET
GWIYDMENESDKYYFNPETKKACKGINLIDDIKYYFDEKGIMRTGLISFENNNYYFNENGE
MQFGYINIEDKMFYFGEDGVMQIGVFNTPDGFKYFAHQNTLDENFEGESINYTGWLDLDEK
RYYFTDEYIAATGSVIIDGEEYYFDPDTAQLVISELEHHHHHH

In a preferred embodiment of the present invention, the lipidated protein is a lipidated OspA heterodimer, particularly the protein of SEQ ID NO: 1 (Lip-S1D1-S2D1), the protein of SEQ ID NO: 2 (Lip-S4D1-S3hybD1) or the protein of SEQ ID NO: 3 (Lip-S5D1-S6D1) or an immunogenic variant thereof with a sequence identity of 60%, 70%, 80%, 85%, 90%, 95%, 96%, 97%, 98%, 99% to SEQ ID NO: 1, 2 or 3. In one embodiment, the composition comprises 3 heterodimers, preferably Lip-S1D1-S2D1 (SEQ ID NO: 1), Lip-S4D1-S3hybD1 (SEQ ID NO: 2) and Lip-S5D1-S6D1 (SEQ ID NO: 3) at a weight ratio of 1:2:1, 1:3:1, 1:1:2, 1:1:3, 1:2:2, 1:2:3, 1:3:2, 1:3:3, 2:1:1, 2:1:2, 2:1:3, 2:2:3, 2:2:1, 2:3:1, 2:3:2, 2:3:3, 3:1:1, 3:1:2, 3:1:3, 3:2:1, 3:2:2, 3:2:3, 3:3:1, or 3:3:2. Preferably, the composition comprises the heterodimer proteins in a weight ratio of 1:1:1 (Lip-S1D1-S2D1 : Lip-S4D1-S3hybD1 : LipS5D1-S6D1).

Sequence identity is frequently measured in terms of percentage identity: the higher the percentage, the more identical the two sequences are. Homologs, orthologs, or variants of a polypeptide will possess a relatively high degree of sequence identity when aligned using standard methods. Methods of alignment of sequences for comparison are well known in the art. Various programs and alignment algorithms are described in: Smith & Waterman (Adv. Appl. Math. 2:482, 1981); Needleman & Wunsch (Mol. Biol. 48:443, 1970); Pearson & Lipman (Proc. Natl. Acad. Sci. USA 85:2444, 1988); Higgins & Sharp (Gene, 73:237-44, 1988); Higgins & Sharp (CABIOS 5: 151-3, 1989); Corpet et al. (Nuc. Acids Res. 16: 10881-90, 1988); Huang et al. (Computer Appls in the Biosciences 8: 155-65, 1992); Pearson et al. (Meth. Mol. Bio. 24:307-31, 1994) and Altschul et al. (J. Mol. Biol. 215:403-10, 1990), presents a detailed consideration of sequence alignment methods and homology calculations. Once aligned, the number of matches is determined by counting the number of positions where an identical nucleotide or amino acid residue is present in both sequences. The percent sequence identity is determined by dividing the number of matches either by the length of the sequence set forth in the identified sequence, or by an articulated length (such as 100 consecutive nucleotides or amino acid residues from a sequence set forth in an identified sequence), followed by multiplying the resulting value by 100. Preferably, the percentage sequence identity is determined over the full length of the sequence. For example, a peptide sequence that has 1166 matches when aligned with a test sequence having 1554 amino acids is 75.0 percent identical to the test sequence (1166÷1554* 100=75.0). The percent sequence identity value is rounded to the nearest tenth. For example, 75.11, 75.12, 75.13, and 75.14 are rounded down to 75.1, while 75.15, 75.16, 75.17, 75.18, and 75.19 are rounded up to 75.2. The length value will always be an integer.

The NCBI Basic Local Alignment Search Tool (BLAST) (Altschul et al. 1990. Mol. Biol. 215:403) is available from several sources, including the National Center for Biotechnology Information (NCBI, Bethesda, MD) and on the internet, for use in connection with the sequence analysis programs BLASTP, BLASTN, BLASTX, TBLASTN and TBLASTX. A description of how to determine sequence identity using this program is available on the NCBI website on the internet. The BLAST and the BLAST 2.0 algorithm are also described in Altschul et al. (Nucleic Acids Res. 25: 3389-3402, 1977). Software for performing BLAST analyses is publicly available through the National Center for Biotechnology Information (ncbi.nlm.nih.gov). The BLASTN program (for nucleotide sequences) uses as defaults a word length (W) of 11, alignments (B) of 50, expectation (E) of 10, M=5, N=-4, and a comparison of both strands. The BLASTP program (for amino acid sequences) uses as defaults a word length (W) of 3, and expectation (E) of 10, and the BLOSUM62 scoring matrix (see Henikoff & Henikoff 1992. Proc. Natl. Acad. Sci. USA 89: 10915- 10919).

Variants of a protein are typically characterized by possession of at least about 60%, for example at least about 70%, , 80%, 85%, 90%, 91%, 92%, 93%, 94%, 95%, 96%, 97%, 98% or 99% sequence identity counted over at least defined number of amino acid residues of the reference sequence, over the full length of the reference sequence or over the full length alignment with the reference amino acid sequence of interest. Proteins with even greater similarity to the reference sequences will show increasing percentage identities when assessed by this method, such as at least 80%, at least 85%, at least 90%, at least 95%, at least 98%, or at least 99% sequence identity. For sequence comparison of nucleic acid sequences, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters are used.

One example of a useful algorithm is PILEUP. PILEUP uses a simplification of the progressive alignment method of Feng & Doolittle {Mol. Evol. 35: 351-360, 1987). The method used is similar to the method described by Higgins & Sharp ( CABIOS 5: 151-153, 1989). Using PILEUP, a reference sequence is compared to other test sequences to determine the percent sequence identity relationship using the following parameters: default gap weight (3.00), default gap length weight (0.10), and weighted end gaps. PILEUP can be obtained from the GCG sequence analysis software package, e.g., version 7.0 (Devereaux et al. 1984. Nuc. Acids Res. 12: 387-395).

As used herein, reference to "at least 80% identity" refers to "at least 80%, at least 85%, at least 90%, at least 91%, at least 92%, at least 93%, at least 94%, at least 95%, at least 96%, at least 97%, at least 98%, at least 99%, or even 100% identity" to a specified reference sequence, e.g. to at least 50, 100, 150, 250, 500 amino acid residues of the reference sequence or to the full length of the sequence. As used herein, reference to "at least 90% identity" refers to "at least 90%, at least 91%, at least 92%, at least 93%, at least 94%, at least 95%, at least 96%, at least 97%, at least 98%, at least 99%, or even 100% identity" to a specified reference sequence, e.g. to at least 50, 100, 150, 250, 500 amino acid residues of the reference sequence or to the full length of the sequence.

An immunogenic variant can induce neutralizing antibodies recognizing the native protein of the pathogen in question.

In another preferred embodiment of the present invention, the lipidated protein is a lipidated *Clostridium difficile* toxin protein, particularly a lipidated form of a protein comprising the *Clostridium difficile* toxin A protein of SEQ ID NO: 13 (Lip-ToxA-His) and/or the *Clostridium difficile* toxin B protein of SEQ ID NO: 14 (Lip-ToxB-His) or an immunogenic variant thereof with a sequence identity of 60%, 70%, 80%, 85%, 90%, 95%, 96%, 97%, 98%, 99% to SEQ ID NO: 13 or 14. The lipidated proteins may be comprised in a composition comprising the proteins in a weight ratio of 1:1 (Lip-ToxA-His : Lip-ToxB-His).

In another preferred embodiment of the present invention, the lipidated protein is a lipidated *Clostridium difficile* toxin protein, particularly a lipidated form of a protein comprising the protein of SEQ ID NO: 7 (C-TAB.G5) and/or a lipidated form of a protein comprising the protein of SEQ ID NO: 8 (C-TAB.G5.1), ), especially the protein of SEQ ID NO: 12 (Lip-C-TAB.G5.1), or an immunogenic variant thereof with a sequence identity of 60%, 70%, 80%, 85%, 90%, 95%, 96%, 97%, 98%, 99% to SEQ ID NO: 7, 8 or 12. The lipidated proteins may be comprised in a composition comprising the proteins in a weight ratio of 1:1 (C-TAB.G5 : C-TAB.G5.1).

In another preferred embodiment of the present invention, the lipidated protein is a lipidated SARS-CoV-2 Spike protein, particularly a lipidated form of a protein comprising the protein of SEQ ID NO: 15 or an immunogenic variant thereof with a sequence identity of 60%, 70%, 80%, 85%, 90%, 95%, 96%, 97%, 98%, 99% to SEQ ID NO: 15.

LIP-Protein\S_2019-nCoV/Italy-INMI1 SEQ ID NO: 15
(Sprotein_hCoV19ItalyINMI1isl2020)

LipCSSMFVFLVLLPLVSSQCVNLTTRTQLPPAYTNSFTRGVYYPDKVFRSSVLHSTQDLF
LPFFSNVTWFHAIHVSGTNGTKRFDNPVLPFNDGVYFASTEKSNIIRGWIFGTTLDSKTQS

LLIVNNATNVVIKVCEFQFCNDPFLGVYYHKNNKSWMESEFRVYSSANNCTFEYVSQPFLM
DLEGKQGNFKNLREFVFKNIDGYFKIYSKHTPINLVRDLPQGFSALEPLVDLPIGINITRF
QTLLALHRSYLTPGDSSSGWTAGAAAYYVGYLQPRTFLLKYNENGTITDAVDCALDPLSET
KCTLKSFTVEKGIYQTSNFRVQPTESIVRFPNITNLCPFGEVFNATRFASVYAWNRKRISN
CVADYSVLYNSASFSTFKCYGVSPTKLNDLCFTNVYADSFVIRGDEVRQIAPGQTGKIADY
NYKLPDDFTGCVIAWNSNNLDSKVGGNYNYLYRLFRKSNLKPFERDISTEIYQAGSTPCNG
VEGFNCYFPLQSYGFQPTNGVGYQPYRVVVLSFELLHAPATVCGPKKSTNLVKNKCVNFNF
NGLTGTGVLTESNKKFLPFQQFGRDIADTTDAVRDPQTLEILDITPCSFGGVSVITPGTNT
SNQVAVLYQDVNCTEVPVAIHADQLTPTWRVYSTGSNVFQTRAGCLIGAEHVNNSYECDIP
IGAGICASYQTQTNSPRRARSVASQSIIAYTMSLGAENSVAYSNNSIAIPTNFTISVTTEI
LPVSMTKTSVDCTMYICGDSTECSNLLLQYGSFCTQLNRALTGIAVEQDKNTQEVFAQVKQ
IYKTPPIKDFGGFNFSQILPDPSKPSKRSFIEDLLFNKVTLADAGFIKQYGDCLGDIAARD
LICAQKFNGLTVLPPLLTDEMIAQYTSALLAGTITSGWTFGAGAALQIPFAMQMAYRFNGI
GVTQNVLYENQKLIANQFNSAIGKIQDSLSSTASALGKLQDVVNQNAQALNTLVKQLSSNF
GAISSVLNDILSRLDKVEAEVQIDRLITGRLQSLQTYVTQQLIRAAEIRASANLAATKMSE
CVLGQSKRVDFCGKGYHLMSFPQSAPHGVVFLHVTYVPAQEKNFTTAPAICHDGKAHFPRE
GVFVSNGTHWFVTQRNFYEPQIITTDNTFVSGNCDVVIGIVNNTVYDPLQPELDSFKEELD
KYFKNHTSPDVDLGDISGINASVVNIQKEIDRLNEVAKNLNESLIDLQELGKYEQYIKWPW
YIWLGFIAGLIAIVMVTIMLCCMTSCCSCLKGCCSCGSCCKFDEDDSEPVLKGVKLHYT

In another preferred embodiment of the present invention, the protein in the vaccine is a hMPV protein, particularly a hMPV F protein, especially in lipidated form, or an immunogenic variant thereof with a sequence identity of 60%, 70%, 80%, 85%, 90%, 95%, 96%, 97%, 98%, 99% to SEQ ID NOs: 16-22. Further information on the hMPV protein is derivable from WO 2020/234300 A1.
**L7F_A1_23 protein sequence SEQ ID NO: 16** 
MSWKVVIIFSLLITPQHGLKESYLEESCSTITEGYLSVLRTGWYTNVFTLEVGDVENLTCA
DGPSLIKTELDLTKSALRELRTVSADQLAREEQIEQPRQSGCGAGATAGVAIAKTIRLESE
VTAIKNALKKTNEAVSTLGNGVRVLATAVRELKDFVSKNLTRAINKNKCDIADLKMAVSFS
QFNRRFLNVVRQFSDNAGITPAISLDLMTDAELARAVSNMPTSAGQIKLMLENRAMVRRKG
FGFLIGVYGSSVIYMVQLPIFGVIDTPCWIVKAAPSCSEKKGNYACLLREDQGWYCQNAGS
TVYYPNEKDCETRGDHVFCDTCAGINVAEQSKECNINISTTNYPCKVSTGRHPISMVALSP
LGALVACYKGVSCSIGSNRVGIIKQLNKGCSYITNQDADTVTIDNTVYQLSKVEGEQHVIK
GRPVSSSFDPVKFPEDQFNVALDQVFESIENSQALVDQSNRILSAGYIPEAPRDGQAYVRK
DGEWVLLSTFLGGLVPRGSHHHHHHSAWSHPQFEK
**sF_A1_K_L7 protein sequence SEQ ID NO: 17** 
MSWKVVIIFSLLITPQHGLKESYLEESCSTITEGYLSVLRTGWYTNVFTLEVGDVENLTCA
DGPSLIKTELDLTKSALRELRTVSADQLAREEQIEQPRQSGCGAGATAGVAIAKTIRLESE
VTAIKNALKKTNEAVSTLGNGVRVLAFAVRELKDFVSKNLTRALNKNKCDIADLKMAVSFS
QFNRRFLNVVRQFSDNAGITPAISLDLMTDAELARAVSNMPTSAGQIKLMLENRAMVRRKG
FGFLIGVYGSSVIYMVQLPIFGVIDTPCWIVKAAPSCSEKKGNYACLLREDQGWYCQNAGS
TVYYPNEKDCETRGDHVFCDTCAGINVAEQSKECNINISTTNYPCKVSTGRHPISMVALSP
LGALVACYKGVSCSIGSNRVGIIKQLNKGCSYITNQDADTVTIDNTVYQLSKVEGEQHVIK
GRPVSSSFDPVKFPEDQFNVALDQVFESIENSQALVDQSNRILSSAESAIGGYIPEAPRDG
QAYVRKDGEWVLLSTFLGGLVPRGSHHHHHHSAWSHPQFEK
**L7F_A1_31 protein sequence SEQ ID NO: 18** 
MSWKVVIIFSLLITPQHGLKESYLEESCSTITEGYLSVLRTGWYTNVFMLEVGDVENLTCA
DGPSLLKTELDLTKSALRNLRTVSADQLAREEQIEQPRQSGCGAGATAGVAIAKTIRLESE
VTAIKNALKKTNEAVSTLGNGVRVLATMVRELKDFVSKNLTRAINKNKCDIADLKMAVSFS
QFNRRFLNVVRQFSDNAGITPAISLDLMTDAELARAVSNMPTSAGQIKLMLENRAMVRRKG
FGILIGVYGSSVIYMVQLPIFGVIDTPCWIVKAAPSCSEKKGNYACLLREDQGWYCQNAGS
TVYYPNEKDCETRGDHVFCDTCAGINVAEQSKECNINISTTNYPCKVSTGRHPISMVALSP
LGALVACYKGVSCSIGSNRVGIIKQLNKGCSYITNQDADTVTIDNTVYQLSKVEGEQHVIK
GRPVSSSFDPVKFPEDQFNVALDQVFESIENSQALVDQSNRILSAGYIPEAPRDGQAYVRK
DGEWVLLSTFLGGLVPRGSHHHHHHSAWSHPQFEK
**L7F_A1_33 protein sequence SEQ ID NO: 19** 
MSWKVVIIFSLLITPQHGLKESYLEESCSTITEGYLSVLRTGWYTNVFMLCVGDVENLTCA
DGPSLLKTELDLTKSALRELRTVSADQLAREEQIEQPRQSGCGAGATAGVAIAKTIRLESE
VTAIKNALKKTNEAVSTLGNGVRVLATMVRELCDFVSKNLTRAINKNKCDIADLKMAVSFS
QFNRRFLNVVRQFSDNAGITPAISLDLMTDAELARAVSNMPTSAGQIKLMLENRAMVRRKG
FGFLIGVYGSDVIYMVQLPIFGVIDTPCWIVKAAPSCSEKKGNYACLLREDQGWYCQNAGS
TVYYPNEKDCETRGDHVFCDTCAGINVAEQSKECNINISTTNYPCKVSTGRHPISMVALSP
LGALVACYKGVSCSIGSNRVGIIKQLNKGCSYITNQDADTVTIDNTVYQLSKVEGEQHVIK
GRPVSSSFDPVKFPEDQFNVALDQVFESIENSQALVDQSNRCCSAGYIPEAPRDGQAYVRK
DGEWVLLSTFLGGLVPRGSHHHHHHSAWSHPQFEK
**L7F_A1_4.2 protein sequence SEQ ID NO: 20** 
MSWKVVIIFSLLITPQHGLKESYLEESCSTITEGYLSVLRTGWYTNVFMLEVGDVENLTCA
DGPSLIKTELDLTKSALRELRTVSADQLAREEQIEQPRQSGCGAGATAGVAIAKTIRLESE
VTAWKNALKKTNEVVSTLGNGVRVLVTMVRELKDFVSKNLTRALNKNKCDIADLKMAVSFS
QFNRRFLNVVRQFSDNAGITPAISLDLMTDAELARAVSNMPTSAGQIKLMLENRAMVRRKG
FGFLIGVYGSSVIYMVQLPIFGVIDTPCWIVKAAPSCSEKKGNYACLLREDQGWYCQNAGS
TVYYPNEKDCETRGDHVFCDTCAGINVAEQSKECNINISTTNYPCKVSTGRHPISMVALSP
LGALVACYKGVSCSIGSNRVGIIKQLNKGCSYITNQDADTVTIDNTVYQLSKVEGEQHVIK
GRPVSSSFDPVKFPEDQFNVALDQVFESIENSQALVDQSNRILSSAESAIGGYIPEAPRDG
QAYVRKDGEWVLLSTFLGGLVPRGSHHHHHHSAWSHPQFEK
**sF_A1_K-E294 protein sequence SEQ ID NO: 21 with substitutions A113C, A339C, T160F, I177L and trimerization helper KLL** 
MSWKVVIIFSLLITPQHGLKESYLEESCSTITEGYLSVLRTGWYTNVFTLEVGDVENLTCA
DGPSLIKTELDLTKSALRELRTVSADQLAREEQIENPRQSRFVLGAIALGVCTAAAVTAGV
AIAKTIRLESEVTAIKNALKKTNEAVSTLGNGVRVLAFAVRELKDFVSKNLTRALNKNKCD
IADLKMAVSFSQFNRRFLNVVRQFSDNAGITPAISLDLMTDAELARAVSNMPTSAGQIKLM
LENRAMVRRKGFGFLIGVYGSSVIYMVQLPIFGVIDTPCWIVKAAPSCSEKKGNYACLLRE
DQGWYCQNAGSTVYYPNEKDCETRGDHVFCDTACGINVAEQSKECNINISTTNYPCKVSTG
RHPISMVALSPLGALVACYKGVSCSIGSNRVGIIKQLNKGCSYITNQDADTVTIDNTVYQL
SKVEGEQHVIKGRPVSSSFDPVKFPEDQFNVALDQVFESIENSQALVDQSNRILSSAESAI
GGYIPEAPRDGQAYVRKDGEWVLLSTFLGGLVPRGSHHHHHHSAWSHPQFEK
**sF_A1_MFur protein sequence SEQ ID NO: 22 with deletion of amino acids at positions 103 to 111, replacement of R102 by a furin site KKRKRR and the substitution G294E, stabilized in post-fusion conformation**

MSWKVVIIFSLLITPQHGLKESYLEESCSTITEGYLSVLRTGWYTNVFTLEVGDVENLTCA
DGPSLIKTELDLTKSALRELRTVSADQLAREEQIENPRQSKKRKRRVATAAAVTAGVAIAK
TIRLESEVTAIKNALKKTNEAVSTLGNGVRVLATAVRELKDFVSKNLTRAINKNKCDIADL
KMAVSFSQFNRRFLNVVRQFSDNAGITPAISLDLMTDAELARAVSNMPTSAGQIKLMLENR
AMVRRKGFGFLIGVYGSSVIYMVQLPIFGVIDTPCWIVKAAPSCSEKKGNYACLLREDQGW
YCQNAGSTVYYPNEKDCETRGDHVFCDTAAGINVAEQSKECNINISTTNYPCKVSTGRHPI
SMVALSPLGALVACYKGVSCSIGSNRVGIIKQLNKGCSYITNQDADTVTIDNTVYQLSKVE
GEQHVIKGRPVSSSFDPVKFPEDQFNVALDQVFESIENSQALVDQSNRILSSAEKGNTSGR
ENLYFQGGGGSGYIPEAPRDGQAYVRKDGEWVLLSTFLGGIEGRHHHHHH

The vaccine protein may be encompassed in a pharmaceutical composition. The composition is pharmaceutically acceptable, which allows for administration to a human.

In accordance with the above and in a second aspect, the present invention relates to a pharmaceutical composition comprising the protein of SEQ ID NO: 1 (Lip-S1D1-S2D1), the protein of SEQ ID NO: 2 (Lip-S4D1-S3hybD1) and/or the protein of SEQ ID NO: 3 (LipS5D1-S6D1) or an immunogenic variant thereof with a sequence identity of 60%, 70%, 80%, 85%, 90%, 95%, 96%, 97%, 98%, 99% to SEQ ID NOs: 1, 2 or 3, particularly comprising the proteins in a weight ratio of 1:1:1 (Lip-S1D1-S2D1 : Lip-S4D1-S3hybD1 : Lip-S5D1-S6D1),
in which about 40-60 % of the fatty acids are palmitic acid, about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms; or
in which 23±10% of the total lipidated proteins comprise two palmitic acids (16: 0) and one C16:1 fatty acid, 41±10% of the total lipidated proteins comprise two palmitic acids (16: 0) and one and one C17:1 fatty acid, 25±10% of the total lipidated proteins comprise two palmitic acids (16: 0) and one C18:1 fatty acid and 12±10% of the total lipidated proteins comprise two palmitic acids (16: 0) and one cycC19 fatty acid, or
in which 18±10% of the total lipidated proteins comprise two palmitic acids (16: 0) and one C16:1 fatty acid, 46±10% of the total lipidated proteins comprise two palmitic acids (16: 0) and one and one C17:1 fatty acid, 20±10% of the total lipidated proteins comprise two palmitic acids (16: 0) and one C18:1 fatty acid and 16±10% of the total lipidated proteins comprise two palmitic acids (16: 0) and one cycC19 fatty acid.

In accordance with the above and in a third aspect, the present invention relates to a pharmaceutical composition comprising the lipidated form of a protein comprising the protein of SEQ ID NO: 7 (C-TAB.G5) and/or a lipidated form of a protein comprising the protein of SEQ ID NO: 8 (C-TAB.G5.1), especially the protein of SEQ ID NO: 12 (Lip-C-TAB.G5.1), and/or the protein of SEQ ID NO: 13 (Lip-ToxA-His) and/or the *Clostridium difficile* toxin B protein of SEQ ID NO: 14 (Lip-ToxB-His), or an immunogenic variant thereof with a sequence identity of 60%, 70%, 80%, 85%, 90%, 95%, 96%, 97%, 98%, 99% to SEQ ID NOs: 7, 8, 12, 13 or 14,
in which about 40-60 % of the fatty acids are palmitic acid, about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms; or
in which 23±10% of the total lipidated proteins comprise two palmitic acids (16: 0) and one C16:1 fatty acid, 41±10% of the total lipidated proteins comprise two palmitic acids (16: 0) and one and one C17:1 fatty acid, 25±10% of the total lipidated proteins comprise two palmitic acids (16: 0) and one C18:1 fatty acid and 12±10% of the total lipidated proteins comprise two palmitic acids (16: 0) and one cycC19 fatty acid, or
in which 18±10% of the total lipidated proteins comprise two palmitic acids (16: 0) and one C16:1 fatty acid, 46±10% of the total lipidated proteins comprise two palmitic acids (16: 0) and one and one C17:1 fatty acid, 20±10% of the total lipidated proteins comprise two palmitic acids (16: 0) and one C18:1 fatty acid and 16±10% of the total lipidated proteins comprise two palmitic acids (16: 0) and one cycCl9 fatty acid.

In accordance with the above and in a forth aspect, the present invention relates to a pharmaceutical composition comprising a protein comprising the protein of SEQ ID NO: 15 (Spike protein) or an immunogenic variant thereof with a sequence identity of 60%, 70%, 80%, 85%, 90%, 95%, 96%, 97%, 98%, 99% to SEQ ID NO: 15,
in which about 40-60 % of the fatty acids are palmitic acid, about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms; or
in which 23±10% of the total lipidated proteins comprise two palmitic acids (16: 0) and one C16:1 fatty acid, 41±10% of the total lipidated proteins comprise two palmitic acids (16: 0) and one and one C17:1 fatty acid, 25±10% of the total lipidated proteins comprise two palmitic acids (16: 0) and one C18:1 fatty acid and 12±10% of the total lipidated proteins comprise two palmitic acids (16: 0) and one cycC19 fatty acid, or
in which 18±10% of the total lipidated proteins comprise two palmitic acids (16: 0) and one C16:1 fatty acid, 46±10% of the total lipidated proteins comprise two palmitic acids (16: 0) and one and one C17:1 fatty acid, 20±10% of the total lipidated proteins comprise two palmitic acids (16: 0) and one C18:1 fatty acid and 16±10% of the total lipidated proteins comprise two palmitic acids (16: 0) and one cycC19 fatty acid.

In accordance with the above and in a fifth aspect, the present invention relates to a pharmaceutical composition comprising a protein comprising any of SEQ ID NOs: 16-22 (hMPV F protein) or an immunogenic variant thereof with a sequence identity of 60%, 70%, 80%, 85%, 90%, 95%, 96%, 97%, 98%, 99% to SEQ ID NO: 16-22,
in which about 40-60 % of the fatty acids are palmitic acid, about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms; or
in which 23±10% of the total lipidated proteins comprise two palmitic acids (16: 0) and one C16:1 fatty acid, 41±10% of the total lipidated proteins comprise two palmitic acids (16: 0) and one and one C17:1 fatty acid, 25±10% of the total lipidated proteins comprise two palmitic acids (16: 0) and one C18:1 fatty acid and 12±10% of the total lipidated proteins comprise two palmitic acids (16: 0) and one cycC19 fatty acid, or
in which 18±10% of the total lipidated proteins comprise two palmitic acids (16: 0) and one C16:1 fatty acid, 46±10% of the total lipidated proteins comprise two palmitic acids (16: 0) and one and one C17:1 fatty acid, 20±10% of the total lipidated proteins comprise two palmitic acids (16: 0) and one C18:1 fatty acid and 16±10% of the total lipidated proteins comprise two palmitic acids (16: 0) and one cycC19 fatty acid.

In a preferred embodiment, the pharmaceutical composition comprises the protein of any of SEQ ID NO: 1, 2, and/or 3 and/or the lipidated form of a protein comprising the protein of SEQ ID NO: 7 (C-TAB.G5) and/or the lipidated form of a protein comprising the protein of SEQ ID NO: 8 (C-TAB.G5.1), especially the protein of SEQ ID NO: 12 (Lip-C-TAB.G5.1), and/or the protein of SEQ ID NO: 13 (Lip-ToxA-His) and/or the *Clostridium difficile* toxin B protein of SEQ ID NO: 14 (Lip-ToxB-His), and/or a protein comprising the protein of SEQ ID NO: 15 (Spike protein) and/or a protein comprising the protein any of SEQ ID NOs: 16-22 (hMPV F protein), or an immunogenic variant thereof with a sequence identity of 60%, 70%, 80%, 85%, 90%, 95%, 96%, 97%, 98%, 99% to any SEQ ID NOs: 1, 2, 3, 8, 9 and 12-22 produced by the method of the first aspect of the present invention.

The pharmaceutical composition may optionally contain any pharmaceutically acceptable carrier or excipient, such as buffer substances, stabilizers or further active ingredients, especially ingredients known in connection with pharmaceutical compositions and/or vaccine production. The composition may comprise sodium phosphate, sodium chloride, L-methionine, sucrose and Polysorbate-20 (Tween 20) at a pH of 6.7 +/- 0.2. Preferably, the pharmaceutical composition also comprises aluminium hydroxide, preferably at a concentration of 0.15%. Additionally, the composition may comprise between 5 mM and 50 mM sodium phosphate, between 100 and 200 mM sodium chloride, between 5 mM and 25 mM L-methionine, between 2.5% and 10% Sucrose, between 0.01% and 0.1% Tween 20 and between 0.1% and 0.2% (w/v) aluminium hydroxide. More preferably, the formulation comprises 10 mM sodium phosphate, 150 mM sodium chloride, 10 mM L-methionine, 5% Sucrose, 0.05% Tween 20 and 0.15% (w/v) aluminium hydroxide at pH 6.7 ± 0.2.

In a preferred embodiment, the pharmaceutical composition or vaccine further comprises a pharmaceutically acceptable excipient. In a more preferred embodiment, the pharmaceutically acceptable excipient is L-methionine.

The OspA protein may be used for vaccination, particularly against an infection caused by *Borrelia* species, more preferably pathogenic *Borrelia* species as disclosed herein more preferably comprising *B. burgdorferi* s.s., *B. afzelii, B. bavariensis* and *B. garinii,* and/or other pathogens against which the antigens have been included in the vaccine. Preferably, the *Borrelia* species is selected from *B. burgdorferi* s.s., *B. garinii, B. afzelii, B. andersoni, B. bavariensis, B. bissettii, B. valaisiana, B. lusitaniae, B. spielmanii, B. japonica, B. tanukii, B. turdi* or *B. sinica* infection, preferably a *B. burgdorferi* s.s., *B. afzelii, B. bavariensis, B. mayonii* and *B. garinii.*

Also the *Clostridium difficile* toxin protein is used for vaccination, particularly against an infection caused by *Clostridium difficile.*

Also the Spike protein of SARS_CoV-2 protein is used for vaccination, particularly against an infection caused by SARS_COV-2.

Also the hMPV F protein is used for vaccination, particularly against an infection caused by hMPV.

The composition according to the present invention may comprise the vaccine protein and an adjuvant. Any of the vaccines or compositions described herein may be administered to a subject with, prior to, or after administration of one or more adjuvants. An adjuvant is a molecule that enhances a response in a subject, such as an immune response, to an antigen or other molecule. In some embodiments, an adjuvant may stabilize an antigen or other molecule. Examples of adjuvants may include, without limitation, aluminium salt (aluminium hydroxide or aluminium phosphate), calcium phosphate hydroxide, paraffin oil, killed bacteria, bacterial toxins, toxoids, subunits of bacteria, squalene, thimerosal, detergents, IL-1, IL-2, IL-12, 2-component adjuvants, such as 2-component adjuvants containing an antibacterial peptide and a TLR9 agonist *(e.g.,* IC31^{®}), and combinations such as Freund's complete adjuvant and Freund's incomplete adjuvant. In some embodiments, the vaccine or composition disclosed herein is administered with aluminium hydroxide. In some embodiments, the vaccine or composition is administered with aluminium phosphate salt. A preferred aluminium salt is an aluminium hydroxide with reduced Cu content, e.g. lower than 1.25 ppb based on the weight of the composition, an adjuvant described in detail in WO 2013/083726 and Schlegl et al., Vaccine 33 (2015) 5989-5996. The unit ppb (parts per billion) is often used in the field of mass spectrometry to quantify impurities. In case of aqueous solutions, 1 ppb means that 1 ng of substance (impurity) is present in 1 g solution, which means that 1 ppb equals 1 µg/l (assuming that 1 liter of solution has a weight of 1 kg).

Adjuvants typically serve to bring the antigen, the substance that stimulates the specific protective immune response, into contact with the immune system and influence the type of immunity produced, as well as the quality of the immune response (magnitude or duration), Adjuvants can also decrease the toxicity of certain antigens; and provide solubility to some vaccines components. Studies have shown that many aluminium-containing vaccines cause higher and more prolonged antibody responses than comparable vaccines without the adjuvant. The benefit of adjuvants has usually been observed during the initial immunization series rather than with booster doses. There are three general types of aluminium-containing adjuvants: aluminium hydroxide, aluminium phosphate and potassium aluminium sulphate (collectively often referred to as "alum"). To work as an adjuvant, the antigen is typically adsorbed to the aluminium particles; that is, it is complexed with the aluminium salt to keep the antigen at the site of injection.

Aluminum adjuvants have been used in practical vaccination for more than half a century. They induce early, high-titer, long-lasting protective immunity. Billions of doses of aluminium-adjuvanted vaccines have been administered over the years. Their safety and efficacy have made them the most popular adjuvants in vaccines to date. In general, aluminum adjuvants are regarded as safe when used in accordance with current vaccination schedules. In human vaccinations, historically, aluminum adjuvants have been used, e.g., in tetanus, diphtheria, pertussis and poliomyelitis vaccines as part of standard child vaccination programs.

Accordingly, in a preferred embodiment, the pharmaceutical composition further comprises an adjuvant, particularly the adjuvant is an aluminium adjuvant, preferably wherein said aluminium adjuvant is aluminium hydroxide. The aluminium hydroxide may comprise less than 1.25 ppb copper based on the weight of the composition. In another preferred embodiment, pharmaceutical composition further comprises L-methionine as pharmaceutically acceptable excipient.

Any of the pharmaceutical compositions according to the present invention may be used as a medicament, particularly a vaccine. The pharmaceutical composition comprising an OspA protein may be used in a method for eliciting an immune response in a human against Lyme disease, particularly wherein the immune response elicited comprises an anti-OspA serotype 1, an anti-OspA serotype 2, an anti-OspA serotype 3, an anti-OspA serotype 4, anti-OspA serotype 5 and/or an antiOspA serotype 6 antibody response with bactericidal activity and/or particularly wherein the immune response elicited comprises antibodies against borrelia serotypes 1, 2, 3, 4, 5 and 6. The pharmaceutical composition comprising the *Clostridium difficile* protein as defined above may be used in a method for eliciting an immune response in a human against a disease caused by *Clostridium difficile.* The pharmaceutical composition comprising an SARS-CoV-2 protein as defined above may be used in a method for eliciting an immune response in a human against a disease caused by SARS-CoV-2, particularly against COVID-19. The pharmaceutical composition comprising an hMPV protein as defined above may be used in a method for eliciting an immune response in a human against a disease caused by hMPV.

The pharmaceutical compositions according to the invention may be administered to a human subject as an injectable composition, for example as a sterile aqueous dispersion, preferably isotonic. The composition may be administered via a systemic or mucosal route. These administrations may include injection via the intramuscular, intraperitoneal, intradermal or subcutaneous routes; or via mucosal administration to the oral/alimentary, respiratory or genitourinary tracts. Although the vaccine of the invention may be administered as a single dose, components thereof may also be co-administered together at the same time.

In the process of vaccination, a protein, such as an OspA protein or a *Clostridium difficile* toxin protein, may be administered along with an aluminum composition, which is used as an adjuvant. An adjuvant is a substance that acts to accelerate, prolong, or enhance antigen-specific immune responses when used in combination with specific vaccine antigens. Aluminum compounds, particularly aluminum salts, are well-known adjuvants. Aluminum phosphate (AlPO₄), aluminum hydroxide (Al(OH)₃), and other aluminum precipitated vaccines are currently the most commonly used adjuvants with human and veterinary vaccines. The adjuvants are often referred to as "alum" in the literature.

In a preferred embodiment, the vaccine comprises an OspA protein, preferably the heterodimer protein of SEQ ID NO: 1 (Lip-S1D1-S2D1), the heterodimer protein of SEQ ID NO: 2 (Lip-S4D1-S3hybD1) and the heterodimer protein of SEQ ID NO: 3 (Lip-S5D1-S6D1), to be administered
- to a human adult a protein content of said 3 heterodimers in the range of from 120 to 200 µg per dose; or
- to a human child a protein content of said 3 heterodimers in the range of from 60 to 100 µg per dose.

In a preferred embodiment, the vaccine comprises a *Clostridium difficile* toxin protein, particularly the *Clostridium difficile* toxin fusion protein of SEQ ID NO: 7 (C-TAB.G5) and/or the *Clostridium difficile* toxin fusion protein of SEQ ID NO: 8 (C-TAB.G5.1), to be administered to a human at a dose of from 20 to 200 µg.

In another preferred embodiment, the vaccine comprises the heterodimer protein of SEQ ID NO: 1 (Lip-S1D1-S2D1), the heterodimer protein of SEQ ID NO: 2 (Lip-S4D1-S3hybD1) and the heterodimer protein of SEQ ID NO: 3 (Lip-S5D1-S6D1) and is to be administered
- to a human adult at least two times, preferably at least three times at a total protein content of said 3 heterodimers in the range of from 120 to 200 µg per dose; or
- to a human child at least two times, preferably at least three times at a total protein content of said 3 heterodimers in the range of from 60 to 100 µg per dose.

In another preferred embodiment, the vaccine comprises the *Clostridium difficile* toxin fusion protein of SEQ ID NO: 7 (C-TAB.G5) and/or the *Clostridium difficile* toxin fusion protein of SEQ ID NO: 8 (C-TAB.G5.1) to be administered to a human at least three times at a total protein content of said 2 toxin fusion proteins at a dose of from 20 to 200 µg, particularly at a dose of 20 µg at the first administration, at a dose of 75 µg at a second administration and at a dose of 200 µg at a third administration, wherein the second and third administrations are 7 days and 21 days from the first administration, respectively.

The terms "comprising", "comprise" and "comprises" herein are intended by the inventors to be optionally substitutable with the terms "consisting of", "consist of" and "consists of", respectively, in every instance. The term "comprises" means "includes". Thus, unless the context requires otherwise, the word "comprises", and variations such as "comprise" and "comprising" will be understood to imply the inclusion of a stated compound or composition (e.g., nucleic acid, polypeptide, antibody) or step, or group of compounds or steps, but not to the exclusion of any other compounds, composition, steps, or groups thereof. The abbreviation, "e.g." is derived from the Latin exempli gratia, and is used herein to indicate a non-limiting example. Thus, the abbreviation "e.g." is synonymous with the term "for example".

Unless otherwise explained, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1 -56081 -569-8).

The singular terms "a", "an", and "the" include plural referents unless context clearly indicates otherwise. Similarly, the word "or" is intended to include "and" unless the context clearly indicates otherwise. The term "plurality" refers to two or more. It is further to be understood that all base sizes or amino acid sizes, and all molecular weight or molecular mass values, given for nucleic acids or polypeptides are approximate, and are provided for description. Additionally, numerical limitations given with respect to concentrations or levels of a substance, such as an antigen, may be approximate.

The present invention is further illustrated by the following Figures, Tables and Examples, from which further features, embodiments and advantages may be taken. As such, the specific modifications discussed are not to be construed as limitations on the scope of the invention. It will be apparent to the person skilled in the art that various equivalents, changes, and modifications may be made without departing from the scope of the invention, and it is thus to be understood that such equivalent embodiments are to be included herein.

### EXAMPLES

### Example 1. Fermentation process for a reproducible heterodimer lipidation pattern

During the course of development of the multimeric *Borrelia* vaccine, a generic production process was developed which was suitable for production of all three OspA antigen proteins at lab scale (SEQ ID Nos: 1-3). During scale-up, it was observed that the lipidation pattern/profile, e.g., the relative amount of fatty acid species added to the protein during post-translational modification, of the recombinant antigen proteins can be influenced and triggered by fermentation parameters during fed-batch process.

*Fermentation* The process was started from a cryo vial for inoculation of the pre-culture in a shaker flask. In general, the fermentation process consists of inoculation of the synthetic batch medium with the pre-culture, a batch-phase and a feed-phase, during which biomass generation/product formation occurs. The main fermentation is completed in less than 20 h, giving a yield of wet cell biomass of approximately >90 g/L.

The timing of the feed-phase start is determined by glucose exhaustion. An automatic switch from batch-phase to feed-phase due to a dissolved oxygen (DO) spike is preferred. The exponential feed continues for 7 h by controlling the specific growth rate of the culture to 0.2 µ (i.e., *E. coli* doubling time reduced to about 3 h 30 min) by limiting the primary carbon source (glucose). During the exponential feed-phase, product formation is initiated after 4 h by IPTG pulse induction, followed by harvest after 3 h. Throughout the fed-batch process, the dissolved oxygen level (DO) is maintained at a constant level. The process is monitored by in-process controls for several fermenter parameters such as temperature, pH, DO, aeration rate, agitation rate, feeding rate, acid/base consumption and headspace pressure.

The OspA heterodimer proteins have a lipid moiety attached at the N terminus. A schematic picture of a fully tri-lipidated protein is depicted in **Figure 1****,** assuming the lipid moiety comprises three C16:0 fatty residues.

As described in the literature, however, recombinantly produced OspA proteins have a complex and heterogeneous lipidation pattern that can depend on many factors, including the expression host and process conditions (Bouchon et al., 1997, *supra).* For more details about protein lipidation, refer to Nadolski et al., 2017 (FEBS Journal 274:5202-5210). Fatty acid composition may be analyzed by Gas Chromatography with Flame-Ionization Detection (GC-FID). Briefly, after release of the fatty acids from the heterodimers by alkaline hydrolysis (1N KOH) and direct methylation based on a method of O'Fallon et al., 2007 (Anim Sci 85:1511-1521), the methyl esters were subsequently loaded on an Agilent 7890B gas chromatography system with FID. Separation was performed on a J&W HP-88 capillary GC column according to Agilent application note 5990-8429 (Agilent application note 5990-8429: "Analysis of Fatty Acids in Infant formulas Using an Agilent J&W HP-88 Capillary GC Column", Agilent Technologies). The tri-lipidated OspA heterodimers have a characteristic lipidation pattern comprised of fatty acids ranging from C14 to C18 and including cycC19. Saturated fatty acids (C14:0, C16:0) and monounsaturated fatty acids (C16:1, C17:1 & C18:1). C16:0 (~50%) and C17:1 (15-20%) were identified as the major components of the total fatty acid content. The fatty acid distribution of toxicological, engineering and GMP drug substance material, as determined by Gas Chromatography with Flame-Ionization Detection (GC-FID), is shown in **Table A-1.**

Further, an RP-HPLC method was developed to characterize the lipidated heterodimers. This method is capable of separating the desired intact tri-lipidated proteins from di-lipidated and degraded by-products, which are mainly N-terminal lipidated monomers clipped in the linker region (i.e., degradation products). Separation was performed on a Zorbax 300SB-CN column (4.6x150mm, 5µm; Agilent) in a water / acetonitrile gradient (0.1% TFA) from 35 to 60% acetonitrile within 10 minutes (flow rate 1 mL/min, column temperature 80°C). A representative RP-HPLC chromatogram of GMP sample M-154 is depicted in **Figure 2****. Table 1** shows the major peak areas in percentage (%) for toxicological (Tox), engineering (R) and GMP (M) drug substance material of three different lipidated OspA fusion proteins. Based on historical data, the tri-lipidated main peak area is ≥85% and the di-lipidated isoforms are ≤10% (see Fig. 2) with peak P1+2: 20-35% and peak P3: 33-44%. Note: this is not an official specification.

**Table 1. Lipid isoform profile of the tri-lipidated OspA heterodimers from toxicological (Tox), engineering (R) and GMP (M) drug substance material. The RP-HPLC profile of the highlighted M-154 drug substance is shown in Figure 2.**

| **Protein** | **Runs** | **P 1+2** | **P 3** | **P 4** | **P 5+6** |
|---|---|---|---|---|---|
| | | **(%)** | **(%)** | **(%)** | **(%)** |
| Lip-S1D1-S2D1 (SEQ ID NO: 1) | Tox 001 | 34.8 | 22.5 | 32.1 | 10.6 |
| | R-151 | 29.7 | 33.1 | 26.3 | 11.0 |
| | M-151 | 25.4 | 38.7 | 24.1 | 11.7 |
| | 1 | 22 | 41 | 24 | 13 |
| | 2 | 23 | 41 | 22 | 14 |
| | 3 | 21 | 43 | 22 | 15 |
| | 4 | 20 | 46 | 20 | 15 |
| | 5 | 18 | 47 | 19 | 17 |
| Lip-S5D1-S6D1 (SEQ ID NO: 3) | Tox 002 | 30.4 | 35.0 | 26.2 | 8.4 |
| | R-155 | 27.1 | 35.9 | 28.4 | 8.6 |
| | M-155 | 23.2 | 40.8 | 24.3 | 11.7 |
| | 1 | 22 | 43 | 24 | 11 |
| | 2 | 24 | 42 | 23 | 11 |
| | 3 | 18 | 48 | 20 | 14 |
| | 4 | 16 | 48 | 20 | 17 |
| Lip-S4D1-S3hybD1 (SEQ ID NO: 2) | Tox 003 | 29.7 | 33.5 | 26.7 | 10.2 |
| | Tox 004 | 30.5 | 32.9 | 26.1 | 10.5 |
| | R-154 | 27.0 | 32.7 | 29.2 | 11.0 |
| | M-154 | 23.5 | 38.4 | 25.9 | 12.2 |
| | 1 | 21 | 41 | 24 | 14 |
| | 2 | 23 | 40 | 24 | 13 |
| | 3 | 20 | 44 | 22 | 14 |
| | 4 | 14 | 48 | 19 | 19 |

The results of fatty acid composition determined by mass spectrometry (MS) in combination with GC analysis are shown in **Table 2.** Briefly, it was revealed that peak 1+2 is comprised of fatty acids with residues of 2x C16:0 + 1x C16:1, Peak 3 of fatty acids of 2x C16:0 + 1x C17:1, peak 4 of fatty acids of 2x C16:0 + 1x C18:1 and peak 5+6 of fatty acids of 2x C16:0 + 1x cycC19.

**Table 2. Fatty acid composition of the tri-lipidated heterodimer peaks from RP-HPLC based on mass spectrometry and GC analysis.**

| Lipidated Protein (RP-HPLC peak) | Fatty Acid Composition: (MS/GC analysis) | Desired relative composition for DS |
|---|---|---|
| Peak 1+2: | 2x C16:0 + 1x C16:1 | 23 ± 10% * |
| Peak 3: | 2x C16:0 + 1x C17:1 | 41 ± 10%* |
| Peak 4: | 2x C16:0 + 1x C18:1 | 25 ± 10%* |
| Peak 5+6: | 2x C16:0 + 1x cycC19 | 12 ± 10%* |

| | | |
|---|---|---|
| * The percentage deviations (ranges) preferably are within ±9%, ±8%, ±7%, ±6%, most preferably ±5%. | | |

An alternative profile is depicted below in Table 2-1.

| Lipidated Protein (RP-HPLC peak) | Fatty Acid Composition: (MS/GC analysis) | Desired relative composition for DS |
|---|---|---|
| Peak 1+2: | 2x C16:0 + 1x C16:1 | 18 ± 10% * |
| Peak 3: | 2x C16:0 + 1x C17:1 | 46 ± 10%* |
| Peak 4: | 2x C16:0 + 1x C18:1 | 20 ± 10%* |
| Peak 5+6: | 2x C16:0 + 1x cycC19 | 16 ± 10%* |

| | | |
|---|---|---|
| * The percentage deviations (ranges) preferably are within ±9%, ±8%, ±7%, ±6%, most preferably ±5%. | | |

In order to identify critical fermentation parameters which influence the lipidation pattern of the OspA heterodimers, supporting activities were performed addressing the effect of certain parameters on the lipidation pattern like temperature, DO and pH in a partial DoE and the amount of trace element solution or applying headspace pressure. Trace elements, also called micronutrients, encompass any chemical element required by living organisms that is less than 0.1 percent by volume and are usually as part of a vital enzyme (a cell-produced catalytic protein). In **Table 3,** the identified parameters which had an impact on the characteristic lipid pattern are listed. The standard fermentation conditions and the tested range are shown.

**Table 3. Parameters and the range tested on the effect of lipidation pattern of the heterodimers**

| | Standard | Tested range |
|---|---|---|
| Trace Element cocktail (TE) | 1x | |
| | 1/8000 dilution in batch phase medium | 1x - 5x |
| | 1/60 dilution in feed phase medium | |
| pH | 7.0 | 6.7 -7.3 |
| Headspace Pressure | 0 bar | 0 - 1.2 bar |

*The effect of trace element solution (TE)* Under standard fermentation conditions, the amount of trace element solution was used as determined during the optimization process (1x). Trace element solution components and concentrations are shown in **Table A-3.** Increasing amounts of trace element cocktail in the fed-batch process were tested for effects in particular on the lipidation pattern. The increased amount was added in the batch- and feed-medium. At the end of fermentation, an aliquot of the biomass was analyzed by RP-HPLC. Briefly, aliquots of frozen fermentation samples were lysed with BugBuster^{™} and the recombinant antigens were extracted and separated into the lipid phase using triton X-114. Proteins from the lipid phase were subsequently partially purified with hydroxyapatite resins in batch modus and analyzed by RP-HPLC.

**Table A-3. List of compounds used for preparation of Trace element (1x TE) solution (stock solution) and amounts (w/v) per liter.**

| Order | Amount/Liter | | Molecular formula/Substance | Name | MW | Supplier | Cat.# |
|---|---|---|---|---|---|---|---|
| 1 | 1.600 | g/L | FeCl₃.6H₂O | Iron(III)chloride hexaydrate | 270.30 | Merck | 1.03943.0250 |
| 2 | 0.370 | g/L | CoCl₂ 6H₂O | Cobalt(II)chloride hexahydrate | 237.93 | Merck | 1.02539.0100 |
| 3 | 0.127 | g/L | CuCl₂ 2H₂O | Copper(II)-chloride dihydrate | 170.48 | Merck | 1.02733.0250 |
| 4 | 0.200 | g/L | ZnCl₂ | Zinc chloride | 136.29 | Merck | 1.08816.0250 |
| 5 | 0.200 | g/L | Na₂MoO₄.2H₂O | Sodium molybdate dihydrate | 241.95 | Merck | 1.06524.1000 |
| 6 | 0.050 | g/L | H₃BO₃ | Boric acid | 61.83 | Merck | 1.00165.0100 |
| 7 | 16.70 | mL | HCl 6M | Hydrochloride acid | n.a. | Merck | 1.10164.1000 |

Changes in the lipidation pattern were already detectable at a 1.5-fold TE increase. As shown in **Figure 3A**, at 1.5x TE, the P1+2 peak area increased over 30% while peak P4 decreased. In the overlay, the GMP material M-154 is shown. The shifting of the lipidation pattern towards an increased P1+2 area is even more pronounced at 2x TE **(****Fig. 3B****),** but no further increase is observed even at 5x TE **(****Fig. 3C****).** Interestingly, when the increased amount of TE (2x) in the batch medium was reduced to 1x in the feed medium where induction of protein expression occurs, the lipidation pattern was not affected at all as depicted in **Figure 3D****,** suggesting that the influence of additional trace elements on protein lipidation profiles is mainly during the exponential phase.

*The effect of headspace pressure on lipidation pattern* The application of headspace pressure during fermentation is a common practice to facilitate oxygen supply in large-scale fermentation. Lab-scale fermentation was done without the application of headspace pressure. Increased pressure during fermentation can increase yields of recombinant proteins and may be applied in scaled-up fermentation. In this regard, the effect of headspace pressure on the lipidation pattern of the OspA heterodimers was investigated. Fermentation experiments with headspace pressure up to 0.9 bar were carried out and at the end of fermentation aliquots of the biomass were analyzed by RPC-HPLC. It was observed that headspace pressure up to 0.5 bar had no impact on the lipidation pattern. Beginning with the application of 0.7 bar, however, changes in the lipid pattern were observed **(****Fig. 4A****).** Peak P3 increased, whereas P1+2 and P4 decrease in relation to P3. This effect was more pronounced when the headspace pressure was increased to 0.9 bar during feed-phase **(****Fig. 4B****).**

*The effect of pH on lipidation pattern* The impact of pH on the lipidation pattern was tested in the fed-batch process and restricted to the feed phase. The batch phase was carried out under standard conditions with pH of 7.0, temperature of 37°C and DO of 25%. Experiments were carried out at a 1.5 L lab scale. Of the tested parameters, pH had the most significant effect on the intensity peak pattern. As shown in **Figure 5****,** at a slightly acidic pH the peak area of P1+2 and P4 is lower in respect to P3 whereas at a more basic pH, the peak area of P1+2 and P4 is increased.

A representative RP-HPLC chromatogram with an overlay of fermentations performed at pH of 6.7, 7.0 and 7.3 under standard conditions for temperature of 37°C and DO of 25% is shown in **Figure 6****.** This figure clearly illustrates the potential of modulating the lipid pattern by altering pH.

Valneva developed a Borrelia vaccine comprised of three antigens based on modified lipidated OspA mutant fragments serotype 1-6 (heterodimers). A generic production process was developed suitable for production of all three OspA proteins. Finally, the lipidation signal intensity pattern can be influenced and triggered by fermentation parameters during the fed-batch process like addition of antifoam.

Fermentation: The process started from a cryo vial for inoculation of the pre-culture in a shaker flasks. In general, the fermentation process consists of inoculation of the synthetic batch medium with a pre-culture, a batch- and a feed-phase where biomass generation and product formation occurs during feed-phase. The main fermentation (batch phase and feed phase) is completed in less than 20 h with a yield of wet cell biomass of approximately >90 g/L.

The timing to start the feed-phase is defined by glucose exhaustion at end of batch phase with a preferred automatic switch from batch-phase to feed-phase due to DO-spike. The exponential feed continues for 7 h by controlling the specific growth rate of the culture to 0.2 µ due to limiting the primary carbon source glucose. During the exponential feed rate, product formation for 3 h will be initiated by IPTG pulse induction.

Throughout the fed-batch process the dissolved oxygen level (DO) will be maintained at a constant level. The process will be monitored by in-process controls for several fermenter parameters like temperature, pH, DO, aeration rate, agitation rate, feeding rate, acid/base consumption and head space pressure.

The heterodimer proteins have a lipid moiety attached at the N terminus. A schematic picture of a fully tri-lipidated protein ("peptide") is depicted in Figure 1 assuming the lipid moiety consists of only C16:0 fatty residues.

*The effect of anti-foaming agents on lipidation pattern* Formation of foam is an undesired event during aerobic fermentation processes and causes various problems e.g. blocking of gas filters. Serious problems are therefore often encountered in bioprocess industries, particularly when the system has to be aerated and agitated. In order to suppress the formation of foams, it is a common practice to add antifoaming agents to the medium because small amounts of foam can create conditions that may promote cell lysis and this subsequently leads to increased foam development.

In an aerobic fermenter culture several factors contribute to foam formation and hydrodynamic conditions which in turn are influenced by the introduction of gas, the medium composition, the presence of growing cells, the formation of metabolites and surface-active substances.

The knowledge of antifoams and the impact on cells or their products is rather limited. Recently, the influence on cell growth and recombinant protein production have been investigated (Routledge SJ, 2012). The reported results suggest that optimization of antifoam addition and an understanding of the bioprocesses is important and necessary. Currently, no data is available on the impact of antifoam and post-translational modified recombinantly expressed proteins such as lipidated proteins.

*Investigating the impact of antifoam (AF) on lipidation pattern* In order to identify further critical fermentation parameters which may have an influence on the lipidation pattern of recombinantly expressed proteins, the amount of antifoam addition and the time of the addition were studied. The addition of antifoam is considered necessary but not as a fermentation parameter to be investigated extensively. In general, for a bioprocess at production scale a defined amount of antifoam is added to the batch medium but not to the feed medium. Addition of antifoam during feed e.g. exponential feed is triggered only by foam formation and therefore on demand. Different modes of antifoam dosage were tested; for instance, adding a defined amount of antifoam during feed medium preparation, addition of antifoam on demand and addition of antifoam during feed phase by pulse (bolus) addition. The antifoam agent PPG-2000 was used herein to explore the impact on the lipid pattern.

At the end of fermentation, aliquots of biomass were prepared for RP-HPLC analysis in the following way: Aliquots of frozen fermentation samples were lysed with BugBuster^{™} and an aliquot of the total cell extract was used to extract and separate the antigens with Triton X-114 into the lipid phase. Subsequently, proteins from the lipid phase were partially purified with Hydroxyapatite resins in batch modus and analyzed by RP-HPLC.

Results of antifoam addition on demand are shown in **Figure 7****,** with a focus on peak 3 area of the tri-lipidated protein. A clear correlation between the total amount of antifoam added and the peak 3 area was observed. The peak area was more pronounced for conditions where e.g., antifoam is added only in the batch medium with no foam formation during feed phase, resulting in a lower antifoam concentration per volume. The peak 3 area was observed to decrease with higher antifoam concentrations.

Antifoam can interfere and change surface properties such as lipid content; therefore, in the next step, the amount of antifoam was set in relation to the generated biomass for representative fermentation runs as shown in **Figure 8****.** For the fermentation condition AF on demand **(****Fig. 8A****),** the ratio AF per dry biomass (AF/BDM) was not constant during induction time, which is evident towards the end of fermentation. For the fermentation condition of no antifoam addition during feed phase **(****Fig. 8B****),** the (AF/BDM) ratio decreased. Exponential addition of AF in the feed medium ensured a constant ratio of AF/BDM **(****Fig. 8C****).** Pulse (bolus) addition of AF during the feed phase is shown in **(****Fig. 8D****).** The two AF pulse additions (one before induction and the second after induction) kept the ratio of AF/BDM constant throughout the induction time.

Fermentation runs were performed testing the different conditions of AF addition on the lipidation pattern. The AF present in the feed medium was considered the optimal approach to apply AF in a ensure batch-to-batch consistency. In **Table 4,** the results for two lipidated heterodimers are shown for the condition no AF addition in the feed medium and compared to AF addition by exponential feed. The change in the lipidation intensity pattern is consistent for both heterodimers. The expressed lipidated proteins with no AF have a higher content of peak 3 area but lower areas of peak1+2 and peak 4.

**Table 4. Isoform profile of the tri-lipidated OspA heterodimers Lip-S1D1-S2D1 and LipS4D1-S3hybD1 with the peak area in percentage (%) for the condition no AF addition.**

| | Peak1+2 | Peak3 | Peak4 | Peak5+6 |
|---|---|---|---|---|
| Lip-S1D1-S2D1, No AF | 20.8 | 46.5 | 20.1 | 12.3 |
| Lip-S1D1-S2D1, AF exponential feed | 22.4 | 43.0 | 22.6 | 11.8 |
| | | | | |

| | Peak1+2 | Peak3 | Peak4 | Peak5+6 |
|---|---|---|---|---|
| Lip-S4D1-S3hybD1, No AF | 19.1 | 45.1 | 23.2 | 12.7 |
| Lip-S4D1-S3hybD1, AF exponential feed | 22.0 | 41.7 | 24.5 | 11.7 |

In **Table 5,** an example for AF pulse addition with total AF amount which is comparable to the amount as for the exponential feed addition is shown. Results for the lipidated heterodimer Lip-S5D1-S6D1 show a similar lipidation intensity pattern for peak 1+2, P3, P4 and P5+6. In contrast to apply AF in the feed medium that requires constant and vigorous stirring throughout the feed phase, AF pulse addition via bolus is applicable also for large scale processes, which would allow to establish a process with a pre-defined amount of AF similar to the condition of AF by exponential feed.

**Table 5. Isoform profile of the tri-lipidated OspA heterodimers Lip-S5D1-S6D1 with the peak area in percentage (%) comparing addition of AF by pulse (bolus) and exponential feed (total AF amounts are comparable).**

| | Peak1+2 | Peak3 | Peak4 | Peak5+6 |
|---|---|---|---|---|
| Lip-S5D1-S6D1, AF pulse | 22.1 | 44.1 | 23.4 | 10.5 |
| Lip-S5D1-S6D1, AF exponential feed | 20.3 | 45.4 | 22.9 | 11.7 |

The following pages 50 to 56 contain further embodiments.
1. A method of producing a lipidated protein, wherein the lipidated protein has one to three lipids attached to a glycerol and the N-terminal cysteine of the protein, particularly wherein the lipidated protein has one lipid and a glycerol substituted with two lipids attached to the amino group of the N-terminal cysteine of the protein and/or particularly wherein the three acyl residues of the lipids are independently selected from C₁₄₋₂₀ alkyl and/or C₁₄₋₂₀ alkenyl, preferably wherein the lipidated protein has the formula (I): 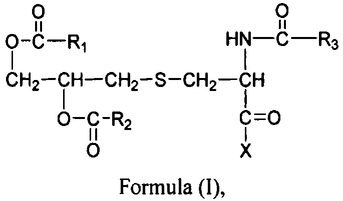 in which R₁, R₂ and/or R₃ are independently selected from C₁₄-C₂₀ alkyl and/or C₁₄-C₂₀ alkenyl and in which X is an amino acid sequence attached to the cysteine residue, the method comprising:
   a) culturing *E coli* cells producing the lipidated protein in a volume of at least 40 L under a defined pressure and a defined pH;
   b) harvesting the produced lipidated protein by extraction from *E. coli* cell culture, with e.g. a detergent, e.g. Triton X-114,

   wherein the pressure and the pH are selected to obtain a lipidation profile of the lipidated proteins, in which about 40- 60 % of the fatty acids are palmitic acid (16:0), about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atomsM; and/or
   wherein the pressure and the pH are selected to obtain an RP-HPLC lipidation profile of the lipidated proteins, wherein a first peak (P1+P2) represents the Lip of formula (I) with two lipids being C16:0 and one being C16:1, a second peak (P3) represents the Lip of formula (I) with two lipids being C16:0 and one being C17:1, a third peak (P4) represents the Lip of formula (I) with two lipids being C16:0 and one being C18:1 and a fourth peak (P5+P6) represents the Lip of formula (I) with two lipids being C16:0 and one being cycC19, wherein peaks P1+P2, P3, P4 and P5+P6 comprise 23±10%, 41±10%, 25±10% and 12±10% of the total lipidated proteins, respectively.
2. The method of item 1, wherein the pressure is a head space pressure of from 0-1 bar, more preferably from 0 to 0.9 bar, even more preferably from 0.4 to 0.9 bar.
3. The method of item 1 or 2, wherein the pH is in the range of about 6.7 to 7.3, more preferably about pH 7.
4. The method of any of items 1 to 3, wherein the volume is at least 100 L, at least 200 L or at least 300 L.
5. The method of any of items 1 to 4, wherein a trace element (TE) solution is added during culturing step a).
6. The method of item 5, wherein said trace element solution comprises Fe, Co, Cu, Zn and/or Mo.
7. The method of item 6, wherein the TE solution comprises 1.6 g/L iron(III)chloride hexahydrate, 0.27 g/L cobalt(II)chloride hexahydrate, 0.127 g/L copper(II)chloride dihydrate, 0.2 g/L zinc chloride, 0.2 g/L sodium molybdate dihydrate, 0.05 g/L boric acid and 16.7 mL hydrochloric acid and is added during culture step a) at a dilution of 1/10000 to 1/10.
8. The method of item 7, wherein the TE solution is added in the batch phase at a dilution in the range of from 1/10000 to 1/1600, such as 1/8000, 1/6400, 1/3200 and 1/1600, preferably 1/8000, and/or wherein the TE solution is added in the batch phase at a dilution in the range of from 1/60 to 1/10, such as 1/60, 1/48, 1/36, 1/24 and 1/12, preferably 1/48 or lower.
9. The method of and of items 1 to 8, wherein an anti-foam agent is present during culturing step a).
10. The method of item 9, wherein anti-foam agent is added during the exponential phase of *E. coli* growth (feed phase).
11. The method of item 10, wherein anti-foam agent is added in a bolus twice during the feed phase, preferably once before induction and once after induction.
12. The method of item 10, wherein anti-foam agent is added continuously during the feed phase (exponentially).
13. The method of any one of items 9 to 12, wherein said anti-foam agent is PPG-2000.
14. The method of any of items 1 to 13,
   a) wherein the lipidated protein is a OspA heterodimer, particularly the protein of SEQ ID NO: 1 (Lip-S1D1-S2D1), the protein of SEQ ID NO: 2 (Lip-S4D1-S3hybD1) or the protein of SEQ ID NO: 3 (Lip-S5D1-S6D1), more particularly is in a composition comprising the proteins in a weight ratio of 1:1:1 (Lip-S1D1-S2D1 : Lip-S4D1-S3hybD1 : Lip-S5D1-S6D1); or
   b) wherein the lipidated protein is a the *Clostridium difficile* toxin A protein of SEQ ID NO: 13 (Lip-ToxA-His) and/or the *Clostridium difficile* toxin B protein of SEQ ID NO: 14 (Lip-ToxB-His), particularly wherein the composition comprises the *Clostridium difficile* toxin fusion proteins in a weight ratio of 1:1, in particular also without the HIS tag; or
   c) wherein the lipidated protein is a *Clostridium difficile* toxin fusion protein of SEQ ID NO: 7 (C-TAB.G5) and/or the *Clostridium difficile* toxin fusion protein of SEQ ID NO: 8 (C-TAB.G5.1); or
   d) wherein the lipidated protein is a SARS_CoV-2 Spike protein of SEQ ID NO: 15; or
   e) wherein the lipidated protein is a hMPV protein of any of SEQ ID NO: 16-22; or
   f) wherein the lipidated protein is an immunogenic variant of any of the lipidated proteins defined in a to e), particularly wherein the variant has sequence identity to any of the proteins of proteins SEQ ID NOs: 1, 2, 3, 7, 8, 13, 14, 15, 16, 17, 18, 19, 20, 21, or 22 of not less than 80%, 85%, 90%, 95%, 96%, 97%, 98%, or 99%.
15. A pharmaceutical composition comprising the protein of SEQ ID NO: 1 (Lip-S1D1-S2D1), the protein of SEQ ID NO: 2 (Lip-S4D1-S3hybD1) and/or the protein of SEQ ID NO: 3 (Lip-S5D1-S6D1), or an immunogenic variant thereof with a sequence identity of 60%, 70%, 80%, 85%, 90%, 95%, 96%, 97%, 98%, 99% to SEQ ID NO: 1, 2 or 3, particularly comprising the proteins in a weight ratio of 1:1:1 (Lip-S1D1-S2D1 : LipS4D1-S3hybD1 : Lip-S5D1-S6D1),
   in which about 40- 60 % of the fatty acids are palmitic acid (16:0), about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms; or
   in which 23±10% of the total lipidated proteins comprise two palmitic acids (16: 0) and one C16:1 fatty acid, 41±10% of the total lipidated proteins comprise two palmitic acids (16: 0) and one and one C17:1 fatty acid, 25±10% of the total lipidated proteins comprise two palmitic acids (16: 0) and one C18:1 fatty acid and 12±10% of the total lipidated proteins comprise two palmitic acids (16: 0) and one cycC19 fatty acid.
16. A pharmaceutical composition comprising a lipidated form of a protein comprising the protein of SEQ ID NO: 7 (C-TAB.G5) and/or a lipidated form of a protein comprising the protein of SEQ ID NO: 8 (C-TAB.G5.1), especially the protein of SEQ ID NO: 12 (Lip-C-TAB.G5.1) and/or the protein of SEQ ID NO: 13 (Lip-ToxA-His) and/or the *Clostridium difficile* toxin B protein of SEQ ID NO: 14 (Lip-ToxB-His), or an immunogenic variant thereof with a sequence identity of 60%, 70%, 80%, 85%, 90%, 95%, 96%, 97%, 98%, 99% to SEQ ID NOs: 7, 8, 12, 13 or 14,
   in which about 40- 60 % of the fatty acids are palmitic acid (16:0), about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms; or
   in which 23±10% of the total lipidated proteins comprise two palmitic acids (16:0) and one C16:1 fatty acid, 41±10% of the total lipidated proteins comprise two palmitic acids (16:0) and one and one C17:1 fatty acid, 25±10% of the total lipidated proteins comprise two palmitic acids (16:0) and one C18:1 fatty acid and 12±10% of the total lipidated proteins comprise two palmitic acids (16:0) and one cycC19 fatty acid.
17. A pharmaceutical composition comprising a lipidated form of a protein comprising the protein of SEQ ID NO: 15 (Spike protein), or an immunogenic variant thereof with a sequence identity of 60%, 70%, 80%, 85%, 90%, 95%, 96%, 97%, 98%, 99% to SEQ ID NO: 15,
   in which about 40-60 % of the fatty acids are palmitic acid, about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms; or
   in which 23±10% of the total lipidated proteins comprise two palmitic acids (16:0) and one C16:1 fatty acid, 41±10% of the total lipidated proteins comprise two palmitic acids (16:0) and one and one C17:1 fatty acid, 25±10% of the total lipidated proteins comprise two palmitic acids (16:0) and one C18:1 fatty acid and 12±10% of the total lipidated proteins comprise two palmitic acids (16:0) and one cycC19 fatty acid.
18. A pharmaceutical composition comprising a lipidated form of a protein comprising any of SEQ ID NOs: 16-22 (hMPV F protein), or an immunogenic variant thereof with a sequence identity of 60%, 70%, 80%, 85%, 90%, 95%, 96%, 97%, 98%, 99% to any of SEQ ID NO: 16-22,
   in which about 40-60 % of the fatty acids are palmitic acid, about 10 to 20 % are mono-unsaturated fatty acids comprising 17 C atoms, about 10 to 20 % are mono-unsaturated fatty acids comprising 18 C atoms, about 5 to 20 % are mono-unsaturated fatty acids comprising 16 C atoms and about 0 to 10% are other fatty acids, particularly in which about 50 % of the fatty acids are palmitic acid, about 10 to 20 % are mono-unsaturated fatty acids comprising 17 C atoms, about 10 to 20 % are mono-unsaturated fatty acids comprising 18 C atoms, about 8 to 15 % are mono-unsaturated fatty acids comprising 16 C atoms and about 1 to 5% are cyclopropane-comprising fatty acids having 19 C atoms; and/or
   in which 23±10% of the total lipidated proteins comprise two palmitic acids (16:0) and one C16:1 fatty acid, 41±10% of the total lipidated proteins comprise two palmitic acids (16:0) and one and one C17:1 fatty acid, 25±10% of the total lipidated proteins comprise two palmitic acids (16:0) and one C18:1 fatty acid and 12±10% of the total lipidated proteins comprise two palmitic acids (16:0) and one cycC19 fatty acid.
19. The pharmaceutical composition according to any of items 15 to 18, wherein the protein of any of SEQ ID NO: 1, 2, and/or 3 and/or the lipidated form of a protein comprising the protein of SEQ ID NO: 7 (C-TAB.G5) and/or the lipidated form of a protein comprising the protein of SEQ ID NO: 8 (C-TAB.G5.1), especially the protein of SEQ ID NO: 12 (Lip-C-TAB.G5.1), and/or the protein of SEQ ID NO: 13 (Lip-ToxA-His) and/or the *Clostridium difficile* toxin B protein of SEQ ID NO: 14 (Lip-ToxB-His), and/or the protein of SEQ ID NO: 15 and/or the lipidated form of a protein comprising nay of SEQ ID NOs: 16-22 (hMPV F protein), or immunogenic variant thereof, particularly wherein the variant has sequence identity to any of the proteins of proteins SEQ ID NOs: 1, 2, 3, 7, 8, 13, 14, 15, 16, 17, 18, 19, 20, 21, or 22 of not less than 80%, 85%, 90%, 95%, 96%, 97%, 98%, or 99%, is produced by the method of any of items 1 to 14.
20. The pharmaceutical composition according to any of items 15 to 19, wherein said composition further comprises an adjuvant.
21. The pharmaceutical composition according to item 20, wherein said adjuvant is an aluminium adjuvant, preferably wherein said aluminium adjuvant is aluminium hydroxide.
22. The pharmaceutical composition according to item 21, wherein said aluminium hydroxide comprises less than 1.25 ppb copper based on the weight of the composition.
23. The pharmaceutical composition according to any of items 15 to 22, wherein said composition further comprises a pharmaceutically acceptable excipient, particularly wherein said pharmaceutically acceptable excipient is L-methionine.
24. The pharmaceutical composition according to any of items 15 to 23 for use as a medicament, particularly a vaccine.
25. The pharmaceutical composition according to any of items 15 to 23 for use in a method for eliciting an immune response in a human
   - against Lyme disease, particularly wherein the immune response elicited comprises an anti-OspA serotype 1, an anti-OspA serotype 2, an anti-OspA serotype 3, an anti-OspA serotype 4, anti-OspA serotype 5 and/or an antiOspA serotype 6 antibody response with bactericidal activity and/or particularly wherein the immune response elicited comprises antibodies against borrelia serotypes 1, 2, 3, 4, 5 and 6; or
   - against a disease caused by *Clostridium difficile*; or
   - against a disease caused by SARS-CoV-2, particularly against COVID-19; or
   - against a disease caused by hMPV.

## Claims

1. A method of producing a lipidated protein, wherein the lipidated protein has the formula (I): 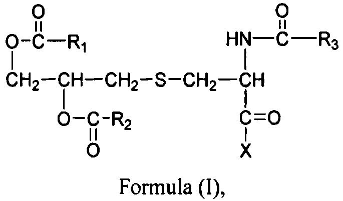 in which R₁, R₂ and/or R₃ are independently selected from C₁₄-C₂₀ alkyl and/or C₁₄-C₂₀ alkenyl and in which X is an amino acid sequence attached to the cysteine residue, the method comprising:
a) culturing *E. coli* cells producing the lipidated protein in a volume of at least 40 L under a defined pressure and a defined pH;
b) harvesting the produced lipidated protein by extraction from *E. coli* cell culture,
wherein the pressure and the pH are selected to obtain an RP-HPLC lipidation profile of the lipidated protein, wherein a first peak (P1+P2) represents the lipid moiety (Lip) of formula (I) with two fatty acids being C16:0 and one being C16:1, a second peak (P3) represents the Lip of formula (I) with two fatty acids being C16:0 and one being C17:1, a third peak (P4) represents the Lip of formula (I) with two fatty acids being C16:0 and one being C18:1 and a fourth peak (P5+P6) represents the Lip of formula (I) with two fatty acids being C16:0 and one being cycC19, wherein peaks P1+P2, P3, P4 and P5+P6 comprise 23±10%, 41±10%, 25±10% and 12±10% of the total lipidated protein, respectively,
wherein said pressure is a head space pressure of from 0.4 to 1 bar and wherein the pH is in the range of 6.7 to 7.3.

2. The method according to claim 1, wherein the headspace pressure is 1 bar.

3. The method according to claim 1 or 2, wherein peaks P1+P2, P3, P4 and P5+P6 comprise 23±5%, 41±5%, 25±5% and 12±5% of the total lipidated protein, respectively.

4. The method according to any one of claims 1 to 3, wherein the pH is about 7, namely wherein the pH is 7 ± 10 % or 7 ± 5 % or 7 ± 2 %.

5. The method according to any one of claims 1 to 4, wherein harvesting step b) is performed with a detergent.

6. The method according to claim 5, wherein the detergent is Triton X-114.

7. The method according to any one of claims 1 to 6, wherein the volume is at least 100 L, at least 200 L, or at least 300 L.

8. The method according to any one of claims 1 to 7, wherein the volume is 1000 L or 2000 L.

9. The method according to any one of claims 1 to 8, wherein a trace element (TE) solution is added during culturing step a), wherein said trace element solution comprises Fe, Co, Cu, Zn and/or Mo.

10. The method according to claim 9, wherein the TE solution comprises 1.6 g/L iron(III)chloride hexahydrate, 0.37 g/L cobalt(II)chloride hexahydrate, 0.127 g/L copper(II)chloride dihydrate, 0.2 g/L zinc chloride, 0.2 g/L sodium molybdate dihydrate, 0.05 g/L boric acid and 16.7 mL/L hydrochloric acid and is added during culturing step a) at a dilution of 1/10000 to 1/10.

11. The method according to claim 10, wherein the TE solution is added in the batch phase at a dilution of 1/10000, 1/8000, 1/6400, 1/3200 or 1/1600 and/or wherein the TE solution is added in the feed phase at a dilution of 1/48, 1/36, 1/24 or 1/12.

12. The method according to any one of claims 1 to 11, wherein an anti-foam agent is present during culturing step a) and wherein
i) the anti-foam agent is added or increased in concentration during the feed phase of *E. coli* growth;
ii) the anti-foam agent is added or increased in concentration by addition of a bolus twice during the feed phase, i.e., once before induction and once after induction; or
iii) the anti-foam agent is added continuously during the feed phase.

13. The method according to claim 12, wherein said anti-foam agent is PPG-2000.

14. The method according to any one of claims 1 to 13,
a) wherein the lipidated protein is an OspA heterodimer, wherein the OspA heterodimer is selected from SEQ ID NO: 1 (Lip-S1D1-S2D1), SEQ ID NO: 2 (LipS4D1-S3hybD1) and SEQ ID NO: 3 (Lip-S5D1-S6D1); or
b) wherein the lipidated protein is the *Clostridium difficile* toxin A protein of SEQ ID NO: 13 (Lip-ToxA-His) and/or the *Clostridium difficile* toxin B protein of SEQ ID NO: 14 (Lip-ToxB-His) with or without the His-tag; or
c) wherein the lipidated protein is a *Clostridium difficile* toxin fusion protein of SEQ ID NO: 7 (C-TAB.G5) and/or the *Clostridium difficile* toxin fusion protein of SEQ ID NO: 8 (C-TAB.G5.1); or
d) wherein the lipidated protein is a SARS-CoV-2 Spike protein of SEQ ID NO: 15; or
e) wherein the lipidated protein is an hMPV protein of any of SEQ ID NOs: 16-22; or
f) wherein the lipidated protein is an immunogenic variant of any of the lipidated proteins defined in a) to e), wherein said immunogenic variant has sequence identity to any of the proteins of SEQ ID NOs: 1, 2, 3, 7, 8, 13, 14, 15, 16, 17, 18, 19, 20, 21, or 22 of not less than 80%, 85%, 90%, 95%, 96%, 97%, 98%, or 99%.

15. The method according to claim 14, wherein
a) the lipidated OspA heterodimers are provided in a composition comprising the proteins in a weight ratio of 1:1:1 (Lip-S1D1-S2D1 : Lip-S4D1-S3hybD1 : LipS5D1-S6D1); or
b) the lipidated *C. difficile* proteins are provided in a composition comprising the *Clostridium difficile* toxin proteins in a weight ratio of 1:1.
